(19) Europäisches Patentamt
European Patent Office
Office européen des brevets

(11) **EP 2 238 553 B1**

(12) **EUROPÄISCHE PATENTSCHRIFT**

(45) Veröffentlichungstag und Bekanntmachung des Hinweises auf die Patenterteilung:
**30.04.2014 Patentblatt 2014/18**

(51) Int Cl.:
***G06F 19/00*** *(2011.01)*    ***G06F 17/30*** *(2006.01)*

(21) Anmeldenummer: **10706147.5**

(22) Anmeldetag: **08.02.2010**

(86) Internationale Anmeldenummer:
**PCT/EP2010/000960**

(87) Internationale Veröffentlichungsnummer:
**WO 2010/089159 (12.08.2010 Gazette 2010/32)**

(54) **SYSTEM FÜR DIE BEREITSTELLUNG INDIVIDUELLER ODER GEPOOLTER AUCH ANONYMISIERTER PATIENTENDATEN AUF GRUNDLAGE VON MOLEKULAREN GENOM-, TRANSKRIPTOM-, PROTEOM-, EPIGENOM ODER METABOLOMDATEN**

SYSTEM FOR MAKING AVAILABLE INDIVIDUAL OR POOLED, ALSO ANONYMOUS PATIENT DATA ON THE BASIS OF MOLECULAR GENOME, TRANSCRIPTOME, PROTEOME, EPIGENOME, OR METABOLOME DATA

SYSTÈME POUR LA MISE À DISPOSITION DE DONNÉES DE PATIENTS INDIVIDUELLES OU GROUPÉES OU ANONYMISÉES SUR LA BASE DE DONNÉES MOLÉCULAIRES GÉNOMIQUES, TRANSCRIPTOMIQUES, PROTÉOMIQUES, ÉPIGÉNOMIQUES OU MÉTABOLOMIQUES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **06.02.2009 US 150383 P**
              **06.02.2009 EP 09075057**

(43) Veröffentlichungstag der Anmeldung:
**13.10.2010 Patentblatt 2010/41**

(73) Patentinhaber: **Cytolon AG**
**10559 Berlin (DE)**

(72) Erfinder: **KLEIN, Thomas**
**14482 Potsdam (DE)**

(74) Vertreter: **Boeckh, Tobias**
**Hertin und Partner**
**Rechts- und Patentanwälte**
**Kurfürstendamm 54/55**
**10707 Berlin (DE)**

(56) Entgegenhaltungen:
WO-A2-2005/097190

• **MAJHAIL N S ET AL: "Double umbilical cord blood transplantation" CURRENT OPINION IN IMMUNOLOGY, ELSEVIER, OXFORD, GB LNKD-DOI:10.1016/J.COI.2006.07.015, Bd. 18, Nr. 5, 1. Oktober 2006 (2006-10-01), Seiten 571-575, XP025079058 ISSN: 0952-7915 [gefunden am 2006-10-01]**
• **INFORMIX: "Informix Guide to SQL - Tutorial - Informix Extended Parallel Server, Version 8.3 Informix - Dynamic Server.2000, Version 9.2" INTERNET CITATION 31. Dezember 1999 (1999-12-31), XP007902863 Gefunden im Internet: URL:http://publib.boulder.ibm.com/epubs/pd f/6530.pdf> [gefunden am 2007-08-30]**
• **BARKER J N ET AL: "Optimizing Unrelated Donor Cord Blood Transplantation", BIOLOGY OF BLOOD AND MARROW TRANSPLANTATION, KLUGE CARDEN JENNINGS PUBLISHING, CHARLOTTESVILLE, VA, US, vol. 15, no. 1, 1 January 2009 (2009-01-01), pages 154-161, XP026064261, ISSN: 1083-8791, DOI: 10.1016/J.BBMT.2008.10.020 [retrieved on 2009-01-13]**

## Beschreibung

Die Erfindung betrifft ein automatisiertes System für die Bereitstellung individueller oder gepoolter und auch anonymisierter Patientendaten auf Grundlage der molekularen Diagnostik auf Ebene der Genoms, des Transkriptoms, des Proteoms, des Epigenoms oder des Metaboloms vor und nach Transplantation oder Therapie von chemischen, biochemischen, biologischen oder Zellprodukten als individuelle oder gepoolte Follow-up Datensammlung auch anonymisiert oder allgemeine oder editierte Datensammlung für die Arzneimittel Entwicklung.

Es bestehen für Knochenmarkregister oder Nabelschnurblutbanken gesetzliche Verpflichtungen die Daten der Patienten, die mit Präparaten aus ihrem Haus transplantiert wurden, bis zu fünf Jahre nach der Transplantation zu erheben und zu sammeln. Die Erhebung der Daten erfolgt heute auf Grundlage von Daten der klinischen Labordiagnostik, die nur eine eingeschränkte Diagnose des Patienten erlaubt. Akademische Organisationen sammeln darüber hinaus diese individuellen Patientendaten, um statistisch relevante wissenschaftliche Aussagen für die regenerative Medizin und die Entwicklung entsprechender Produkte zu liefern.

In den letzten Jahren haben sich verschiedene Verfahren und Methoden herausgebildet, Nabelschnurblut-Präparate (NSB-) zwischen Entnahmezentren oder Nabelschnurblutbanken einerseits und den Kliniken oder Transplantationszentren andererseits zu vermitteln. Alle Verfahren und Methoden haben ihren Ursprung in den Prozessen, die für die Vermittlung von Knochenmark erforderlich sind. Bislang bestehen aber keine automatisierten Prozesse. Eine Klink, die für einen Patienten/Empfänger ein NSB-Präparat für die Transplantation benötigt, fragt bei Registern an, ob sie einen entsprechend verschiedener biologischer und medizinischer Kennungen passendes NSB-Präparat für ihren Patienten haben. Die registrierten Daten können sich beispielsweise auf den so genannten HLA Match beziehen oder auf die im Präparat vorhandene Zellzahl und weitere medizinische bzw. biologische Daten (z.B. Blutgruppe).

Kliniken und Transplantationszentren haben so genannte Koordinatoren, die die Auswahl eines NSB-Transplantates anhand der übermittelten Daten durchführen. Diese Koordinatoren schlagen dem behandelnden Arzt eine Auswahl von Präparaten vor. Der Arzt entscheidet, ob und welches Transplantat genutzt wird. Damit die Kliniken die richtige Nabelschnurblut Einheit bestellen können, muss die Klinik für jedes Präparat alle wesentlichen Daten über das betreffende Präparat erfragen. Für die Informationen, die in einem so genannten Unit Report niedergelegt sind, wurden aber bislang weltweit keine Standards definiert. Auch wurde die Korrelation der Daten einzelner Präparate untereinander bislang nicht erfasst. Koordinatoren unterliegen bei der Präparate Auswahl einem iterativen Prozess, der zeitaufwendig und fehlerbehaftet ist.

Dieses Problem wird dadurch verstärkt, dass viele eingelagerte NSB Präparate, die z.B. nach dem HLA-Match passen würden, für die Transplantation zu klein sind, d.h. dass die Zellzahl des Präparates zu gering ist. Die klinische Forschung der letzten fünf Jahre hat bewiesen, dass die Anzahl der im Nabelschnurblut enthaltenen kernhaltigen Zellen (TC) und nachrangig so genannten CD34+ Zellen (haben die Fähigkeit zur Blutbildung) von entscheidender Bedeutung für den Erfolg der Transplantation ist. Die genetische Passgenauigkeit (HLA-Match) kann bei großer Zellzahl wesentlich geringer sein als vergleichsweise der notwendige HLA-Match bei Knochenmark Transplantationen. Die erforderlichen Zellzahlen und ihre Korrelation zu dem HLA-Match sind zwar im Stand der Technik beschrieben, haben aber noch keinen systematischen Einzug in die etablierten Verfahren gefunden. Koordinatoren und Nabelschnurbanken stehen heute vor der Aufgabe, zueinander passende Präparate in Einzelprüfungen zu identifizieren und mit den Patientendaten zu vergleichen. Dies ist umso schwieriger, da die in Frage kommenden Präparate meist über mehrere Banken verteilt sind und mit verschiedenen Verfahren und Standards beschrieben sind.

Im Stand der Technik, beispielsweise der US 2002/0132343 A1, wird offenbart, dass für die erfolgreiche Behandlung von (Leukämie- etc.) Patienten mit passenden Nabelschnurblutpräparaten eine hohe Zellzahl und eine hohe / garantierte Produktqualität (incl. FDA etc. Zertifizierung) notwendig ist und diese zusätzlich zur einfachen und direkten Verwendung in der Transplantationsklinik zur Verfügung gestellt werden können. Es wird ein Gesamtsystem (SCBS) für expandierte und "gematchte" Stammzellen (nicht nur Nabelschnurblut) beschrieben, welches den gesamten Lebens- und Produktionszyklus umfasst (Quellmaterialgewinnung, Produktion, zertifizierte Qualitätssicherung und Lieferung). Das Gesamtsystem erfüllt die regulatorischen Qualitätsanforderungen und -standards gemäß FDA etc. wie: FACT, CGTP, AAB und aus einem bestehenden (allogenen) Nabelschnurblutvorrat wird ein Präparat ausgewählt, welches zum Patientengewebe passt (Matching). Die US 2002/0132343 A1 beschreibt, dass die bekannten Methoden zur Typisierung (HLA Typisierung von zumindest sechs Loci) der Spender- und Patientenzellen unter Einhaltung der regulatorischen Standards zum Einsatz kommen sollen. Zusätzlich wird beschrieben, dass ein automatisiertes Trackingsystem zur Verfolgung der einzelnen Präparate/Proben sowie zur Nachverfolgung eingesetzt werden kann. Die Spenderzellen (Quellmaterial) stammen aus einer zertifizierten Quelle (Nabelschnurblutbank), die die relevanten Informationen (z.B. TNC, HLA Loci, Anzahl CD34+ Zellen) pro Probe entsprechend den Qualitätsstandards erfasst. Nach der US 2002/0132343 A1 wird das Quellmaterial aufbereitet, so dass nur die relevanten Zellen weiterverarbeitet werden und die gewünschten Zellen (z.B. CD34+ Zellen) werden ex vivo expandiert. Die so produzierten Stammzellprodukte werden als "Patient Treatment Kit" zur Verwendung bereitgestellt. Sie beinhalten dabei eine definierte Charakteristik und sind durch den behandelnden Arzt direkt verwendbar. Ein bestelltes SCBS Produkt matcht mindestens 4/6 Antigene bzw.

3/6 Allele mit $2*10^7$ Zellen/kg bei Kindern (<12J) bzw. Patienten mit <50kg Körpergewicht sowie $1*10^7$ Zellen/kg Körpergewicht bei allen anderen Patienten. Die US 2002/0132343 A1 beschreibt als zentrales Element die Fähigkeit der direkten Lieferung der SCBS-Produkte an die Transplantationszentren, die das Produkt bestellt haben. Hierzu werden die SCBS Produkte in spezielle Container verpackt, die durch Kurierdienste verfrachtet werden. Dabei wird versucht, die Qualitätsstandards aufrecht zu erhalten. Das beschriebene SCBS System und die Methoden betrachten nicht die Problematik der automatisierten Auswahl geeigneter Präparate. Es wird der heutige Stand der Technik zur manuellen Auswahl von Stammzellpräparaten beschrieben (HLA Matching, Zellzahl/Gewichts-Korrelation, etc.). Die US 2002/0132343 A1 zeigt nicht, wie die benötigte Zeit zur Identifikation eines geeigneten Spendermaterials reduziert werden kann bzw. wie die notwendigen manuellen Schritte zur Auswahl zwischen mehreren potentiell geeigneten Präparaten automatisiert werden können.

Weiterhin offenbart die US 2002/0168639 A1, dass es bedingt durch die begrenzte Leistungsfähigkeit der Analysegeräte schwierig ist, eine Gewebeprobe mit einer Vielzahl von Vergleichsproben zu vergleichen. Die US 2002/0168639 A1 beschreibt einen Profilträger, auf dem einerseits ein Probegewebe untergebracht werden kann und andererseits ein Microarray, auf dem unterschiedliche Vergleichsproben zur simultanen Analyse eingesetzt werden können. Die Reaktionsfähigkeit des Testgewebes bzw. der Microarray-Proben werden in einer Datenbank gespeichert und in Beziehung zu anderen Informationen des Patienten, von dem das Testgewebe stammt, gesetzt (z.B. Alter, Gewicht, Geschlecht, Krankheitsgeschichte). Das in der US 2002/0168639 A1 offenbarte Datenbanksystem ist an ein Informations- Management System (IMS) angeschlossen, dass Suchen und Korrelationen durchführen kann. Damit werden Vergleiche und Korrelationen der biologischen Reaktionsfähigkeiten zwischen dem Testgewebe und den Proben des Microarrays erstellt. Hierzu kommen die-Fähigkeiten von State of the Art Business Analytics Produkten zur Datenanalyse und -visualisierung wie bei "Tibco Spotfire" zum Einsatz. Im Rahmen der US 2002/0168639 A1 werden Informationen über Gewebezellen und deren Spender gewonnen und abgespeichert. Diese Informationen werden über Standard Analyseverfahren miteinander verglichen, um Korrelationen etc. für Forschungs- bzw. Diagnosezwecke zu identifizieren. Es wird nicht offenbart, wie die Korrelation im Bezug auf eine konkrete Fragestellung ablaufen soll. Vielmehr wird auf die allgemeinen Möglichkeiten, die dem Stand der Technik entsprechen, verwiesen.

Die GB 2407193 A beschreibt ein System um biologische Zelllinienexperimente mit Bildauswertung automatisiert ablaufen und auswerten zu lassen. Das System besteht einerseits aus einer Einheit, die es ermöglicht neue Experimente zu definieren und automatisiert ablaufen zu lassen, wobei das System dahingehend offen ist, dass beliebige Experimente und Geräte modular registriert und eingesetzt werden können. Die zweite Systemkomponente umfasst die automatisierte Analyse (Bildauswertung) der Experimente - hierbei wird primär auf die Bildauswertung von Assays hingewiesen, d.h. die Ergebnisse der Assays (der Experimente) werden in das System eingespeist und von diesem analysiert. Es können variable/erweiterbare Analysetechniken eingesetzt werden. Das Gesamtsystem steuert selbstständig die Durchführung und die Analyse mehrerer nacheinander folgender Experimente. Der Durchführungsprozess kann hierzu flexibel definiert oder angepasst werden. Die Resultate werden in einer Datenbank abgespeichert und die Ergebnisse über flexibel definierbare Berichte dem Benutzer angezeigt. Die GB 2407193 A zeigt, dass gesamte Laborprozesse für Experimente mit Zelllinien automatisiert werden können. Ähnliches ist aus der industriellen Praxis vieler Anwendungsdomänen bekannt. Die GB 2407193 A gibt ein Rahmengerüst für die automatisierte Datenauswertung im Rahmen des Prozesses vor.

Die US 2004/0121369 A1 adressiert das Problem, den flexiblen Einsatz einer Vielzahl von Geräten und Analysemethoden im Rahmen komplexer biologischer Laborexperimente zu automatisieren. Im Rahmen von sich ändernden Laborprozessen Daten zur Auswertung nacheinander durch unterschiedliche Softwareapplikationen zu schleusen oder die Daten parallel zu verarbeiten, ist aufwändig und bedarf der individuellen menschlichen Koordination (manuelle Datenformatierung oder aufwändige individuelle Programmierung). Die US 2004/0121369 A1 stellt ein flexibles Framework zur Automatisierung von Laborexperimenten und deren Auswertung bereit. Das System ermöglicht die flexible Registrierung (Anbindung) von Laborgerätesteuerungen, um diese im Rahmen von individuell und frei zu definierenden Experimentenabläufen zu verwenden. Ebenso lassen sich Analysegeräte und Analysesoftware flexibel registrieren und in den Gesamtprozess integrieren. Die US 2004/0121369 A1 beschreibt hierfür einen flexiblen Registrierungsmechanismus, der das Problem der unterschiedlichen Schnittstellen und Protokolle der Geräte und Analyseanwendungen löst, so dass diese effizient miteinander gekoppelt werden können. Die Informationen werden in einer Datenbank gespeichert. Die US 2004/0121369 A1 behandelt das Problem der Effizienzsteigerung bei bestimmten Prozessen. Es wird gezeigt, dass eine Aufgabenstellung, die bisher der manuellen Unterstützung durch Fachpersonal bedurfte, gänzlich automatisiert ablaufen kann. Die Problemlösung liegt jedoch im Bereich der effizienten Gerätekopplung und nicht im Bereich der effizienten Auswahl bestimmter Zellpräparate.

Die WO 02/077640 A2 offenbart ein System, um große Datenmengen, die im Zusammenhang mit der Analyse von Biomolekülen mit Hilfe von Microarrays entstehen, effizient aufzubereiten und auszuwerten und gleichermaßen den Analyseprozess zu optimieren. Das offenbarte automatisierte System ermöglicht es, große Datenmengen in einer Datenbank mittels Data Mining Verfahren z.B. nach physikalischen Eigenschaften zu gruppieren und diese Ergebnisse durch ein selbstlernendes neuronales Netz zu analysieren. Mittels mathematischer und statistischer Methoden wird es durch das neuronale Netz ermöglicht, automatisiert neue Proben zu generieren, die eine gewünschte Aufgabenstellung erfüllen. Es werden somit mathematische und statistische (selbstlernende) Algorithmen genutzt, um bestimmte Fragestellungen hinsichtlich eines gesuchten Biomoleküls zu beantworten. Die Algorithmen sind jedoch nicht auf andere Systeme übertragbar, sondern können nur in dem genannten System verwendet werden.

Die US 2008/0014174 A1 beschreibt, die methodische Herstellung von Lymphozytenpräparaten sowie deren Lagerung und ein "Kit" zur fertigen Anwendung beim Patienten. Die Lymphozyten stammen von peripherem Blut von Spendern, die zumindest in 4 Loci mit dem Patienten matchen. Bestimmte Tumore, Virusinfektionen und Autoimmunerkrankungen sollen mittel HLA gematchten allogenen aktivierten Lymphozyten behandelt werden. Es werden keine Aussagen über den genauen Auswahlprozess getätigt.

Weiterhin offenbart die DE 600 30 978 T2 ein Verfahren, welches es ermöglicht, mehrere biologische Proben mittels einer Sensorplattform simultan in einer hochwertigen Art quantitativ zu analysieren. Hierbei werden insbesondere die chemischen und physikalischen Eigenschaften einer zu analysierenden Probe von der Sensorplattform bestimmt und in eine Signalauswertung eingeführt. Das System kann unter anderem zur Bestimmung der HLA Werten von Proben verwendet werden.

WO 2005/097190 beschreibt ein Verfahren zur Bereitstellung von Stammzelldatenbanken für die Auswahl von zur Transplantation geeigneten Stammzellpräparaten.

Im Stand der Technik ist nicht beschrieben, wie genau die Auswahl der Präparate erfolgt. Es ist allgemein bekannt, welche Parameter mindestens zur Auswahl geeigneter Präparate herangezogen werden sollten aber nicht ableitbar, wie aus den analysierten Präparaten auf das "beste" Präparat geschlossen werden kann. Des Weiteren ist im Stand der Technik kein Auswahlsystem beschrieben, dass ein geeignetes Präparat auswählt und dem Koordinator das Ergebnis entsprechend präsentiert und ggf. automatisiert ablaufen kann. Weiterhin ist nicht offenbart, ob eine Beurteilung der Transplantationseffizienz erfolgt. Eine Transplantation muss in regelmäßigen Abständen überwacht werden und es ist im Stand der Technik kein Verfahren oder System offenbart, welches die Überwachung z. B. in einem Computersystem beziehungsweise automatisch durchführen kann.

Zusätzlich werden im Stand der Technik keine Lösungen für den Fall einer Mehrfachtransplantation offenbart. Dies ist eine Lösungsstrategie für den Fall, dass kein geeignet großes Präparat gefunden wird. Dann wird das Suchproblem auf zwei oder mehr Präparate erweitert, die zusammen genügend Zellen beinhalten und zusätzlich sowohl untereinander als auch zum Patienten eine hinreichende Übereinstimmung in den HLA-Werten besitzen.

Aufgabe der Erfindung war es demgemäß, ein System zur Verfügung zu stellen, das nicht die Nachteile des Stands der Technik aufweist, die Auswahl und Verteilung eines geeigneten Präparates ermöglicht und eine Überwachung der Transplantationseffizienz und Beurteilung dieser erlaubt.

Überraschenderweise wird die Aufgabe durch die unabhängigen Ansprüche gelöst. Bevorzugte Ausführungsformen der Erfindung ergeben sich aus den Unteransprüchen.

Es war völlig überraschend, dass ein System für die Vermittlung und Auswahl von biologischen Zellen oder Geweben, insbesondere Nabelschnurblut-Präparaten, für Transplantationen, Therapien und/oder Forschungszwecke zwischen mindestens einem Entnahmezentrum und/oder einer Lagerstätte und mindestens einer Klinik, einem Transplantationszentrum und/oder einer Forschungseinrichtung, wobei diese über verkabelte und/oder kabellose Verbindungen auf einer oder mehreren Verarbeitungseinheiten insbesondere Computer, medizinische Systeme, Speichervorrichtungen und/oder speziellen Prozessoren miteinander kommunizieren und über ein Netzwerk bestehend aus den mehreren Verarbeitungseinheiten, durch welche Daten ausgetauscht werden, miteinander verbunden sind, wobei das System die folgenden Schritte umfasst:

- Eingabe von Erfahrungsdaten der Nabelschnurblut-Präparate in einen Computer und Speicherung auf einem Speichermedium,

- Eingabe von Anfragedaten eines potentiellen Empfängers oder Patienten und Speicherung dieser auf einem Speichermedium,

- Voreinstellung von Suchkriterien, insbesondere der Speicherung der Suchkriterien auf einem Speichermedium und/oder einer Verarbeitungseinheit,

- Patientenrecherche, wobei die Recherche einen Vergleich der Erfahrungsdaten mit den Anfragedaten umfasst und eine automatische Auswertung der Recherche erfolgt und/oder

- anhand dieser Auswertung eine Bestellabwicklung und Nachverfolgung erfolgt und die Bestellung des Nabelschnurblut-Präparates über das Netzwerk erfolgt, wobei

insbesondere die potentiellen Nabelschnurblut-Präparate nach einem HLA-Match, einem Patientengewicht, einer Anzahl der kernhaltigen Zellen (TNC) und einer Anzahl der hämatopoetischen Zellen (CD34+) geordnet und ausgewählt werden, wobei zusätzlich vor und nach einer Transplantation und/oder einer Therapie von biologischen Zellen und/oder Geweben Metabolite der Nabelschnurblut-Präparate, des Empfängers und/oder des Spenders bestimmt werden und Genom-, Proteom-, Transkriptom- und/oder Epigenomanalysen durchgeführt werden, nicht die Nachteile des Stands der Technik aufweist.

Im Sinne der Erfindung beschreibt ein System eine Gesamtheit von technischen Einzelbestandteilen, die aufeinander bezogen sind und wechselwirken. Vorteilhafterweise kann ein System sowohl Programme und DV-Anlagen (Datenverarbeitungsanlagen) sowie Elemente wie Transportbehälter, NSB Präparate umfassen.

Im Sinne der Erfindung beschreiben Verarbeitungseinheiten bevorzugt Eingabeeinheiten, über die Daten oder Informationen eingegeben werden und bevorzugt in digitaler Form gespeichert werden. Die Verarbeitungseinheiten umfassen bevorzugt Computer, medizinische Systeme, Speichervorrichtungen und/oder speziellen Prozessoren, die zur Eingabe und Speicherung geeignet sind. Die Verarbeitungseinheiten können in einer bevorzugten Ausführungsform separat und/oder in verschiedenen Formen von Hardware, Software und/oder Firmware vorliegen. So kann es vorteilhaft sein, wenn medizinische System, wie beispielweise Analysevorrichtungen, die analysierten Daten automatisch in das System übertragen und hierfür keine manuelle Eingabe erforderlich ist.

Die erfindungsgemäße Lehre ist auch eine Kombinationserfindung, bei der die genannten Elemente zusammenwirken und ein System zur Vermittlung und Auswahl eines biologischen Transplantates bereitstellen, dass zudem noch die Transplantation überwacht. Durch das effektive Zusammenwirken der Systemkomponenten, wird ein synergistischer Effekt generiert, der dadurch gekennzeichnet ist, dass ein System überraschenderweise für alle genannten Vorgänge zur Verfügung steht und somit alle Vorgänge von dem System zentral aber auch dezentral überwacht und kontrolliert werden können. Alle an der Transplantation beteiligten Institutionen, umfassend Kliniken, NSB-Banken oder Ärzte können Zugang zu dem System erhalten und den Verlauf der Transplantation verfolgen. Das erfindungsgemäße System, vergleicht die eingehenden Patientendaten oder Anfragedaten mittels einer mehrstufigen Verträglichkeitsmatrix und variierenden Ordnungskriterien mit den Daten oder Erfahrungsdaten registrierter Zellpräparate. Vorteilhafterweise erfolgt der Vergleich vollautomatisch, wobei ein behandelnder Arzt vorteilhafterweise online auf die Daten zugreifen kann. Vorteilhafterweise können dem Arzt automatisch Lösungs-Vorschläge unterbreitet werden, welches Einzel-Präparat (Single-Transplant) oder welche zueinander passenden Präparate (Multi-Transplant) für eine Transplantation in Frage kommen. Damit kann der eigentliche Vorteil der "ready to use" gelagerten NSB-Präparate gegenüber der langwierigen vergleichenden Suche durch Koordinatoren grundlegend verändert und wesentlich verbessert werden. Das System ist für alle biologischen, biochemischen oder chemischen Stoffen geeignet, die einer zeitkritischen Vermittlung bei Transplantationen oder anderen (medizinischen) Anwendungen unterliegen. Zusätzlich zu der Auswahl und Vermittlung eines oder mehrerer Präparate, überwacht das System den Effekt der Transplantation, indem es bevorzugt vor und nach der Transplantation Proben der NSB-Präparate, des Empfängers und/oder des Spenders anfordert, die vorteilhafterweise auf Stoffwechselaktivitäten analysiert werden, d. h. es werden bevorzugt definierte Metabolite, deren Konzentration und/oder deren Präsenz in den Proben bestimmt. Aus diesen Daten können komplexe Modelle der vorliegenden Stoffwechselaktivitäten erstellt werden, die wiederum über den Erfolg der Transplantation entscheiden können. Außerdem kann es vorteilhaft sein, wenn vor und nach der Transplantation Genom-, Proteom-, Transkriptom und/oder Epigenomanalysen durchgeführt werden. Das System ermöglicht eine umfangreiche Darstellung beispielsweise der Reaktionen eines Empfängers auf ein Transplantat, woraus schnell und effizient eine Therapiestrategie abgeleitet werden kann. Es war in diesem Zusammenhang völlig überraschend, dass eine insbesondere automatische Überwachung einer Transplantation und Anforderung von Proben durch das System, zu besseren Transplantationserfolgen führt. Die Proben werden bevorzugt automatisch von dem System angefordert und die resultierenden Daten werden allen Beteiligten, die Zugang zu dem System haben, zur Verfügung gestellt. Überraschenderweise können hierdurch oft durchgeführte Mehrfachuntersuchungen vermieden werden, was wiederum in einer Kostenersparnis resultiert. Außerdem können die vor und nach der Transplantation generierten Daten zur Erhebung von Statistiken genutzt werden, die beispielsweise akademischen Institutionen oder sonstigen Forschungseinrichtungen zur Verfügung gestellt werden können. Anhand dieser Daten können neue Medikamente und optimierte Transplantationsstrategien entwickelt werden, die die bisher geringen Erfolgsaussichten einer Transplantation maßgeblich verbessern können. Das System stellt eine Abkehr von einem bisher üblichen technischen Konzept dar, da die Kliniken oder NSB-Banken zentrale Verwaltungssysteme bevorzugen und keine Vernetzungen der einzelnen System bestehen. Hierdurch kommt es zu einem reduzierten Informationsaustausch, bei dem viele wichtige Informationen zwischen den Beteiligten verloren gehen. Das System stellt somit einen technischen Fortschritt dar und eröffnet einen neuen technischen Bereich.

Es werden charakteristische Erfahrungswerte der NSB-Präparate über Verarbeitungseinheiten, beispielsweise Computer eingegeben. Es kann jedoch auch vorteilhaft sein, wenn ein Präparat automatisch mittels einer oder mehrerer Analysevorrichtungen analysiert wird und untersuchte Werte automatisch in eine Verarbeitungseinheit übertragen werden. Beispielsweise können in sogenannten Laborstraßen, die eine Art Reihenschaltung unterschiedlicher Analysevorrichtungen darstellen, NSB-Präparate schnell und effizient untersucht und charakterisiert werden. Die analysierten Werte werden automatisch in das System eingepflegt und sind so schnell verfügbar.

Die Daten der NSB-Präparate, d. h. die Erfahrungswerte umfassen außerdem molekulardiagnostische Daten, die beispielsweise durch Metabolic Profiling erstellt wurden. Veränderungen im Metabolom geben unmittelbaren Aufschluss über die Reaktionen von Organismen auf genetische Veränderungen und umweltbedingte Einflüsse, weshalb der umfassenden Analyse des Metaboloms eine große Bedeutung bei der Diagnose, Prognostizierung und Klassifizierung von Krankheiten sowie dem Studium der Einflüsse von Toxinen und Medikamenten auf die zelluläre Physiologie zukommt. Das Metabolom bezeichnet im Sinne der Erfindung im Wesentlichen alle charakteristischen Stoffwechsel-Eigenschaften einer Zelle, eines Gewebes oder eines Organs. Die umfassende Charakterisierung aller Metabolite in einem System stellt jedoch hohe Anforderungen an die verwendeten analytischen Methoden, weil Metabolite aus stofflicher Sicht mannigfaltig sind und in einem weiten Konzentrationsbereich vorliegen. Gegenwärtig werden zwei Ansätze für die Untersuchungen des Metaboloms verwendet: Metabolic Profiling und Metabolic Fingerprinting. Metabolic Profiling bezeichnet im Sinne der Erfindung insbesondere die quantitative Bestimmung ausgewählter Metabolite in einem bestimmten Stoffwechselvorgang oder einer Substanzklasse die von biologischem Interesse sind. Dabei werden nicht nur Stoffwechselendprodukte analysiert, sondern auch Schlüsselmetabolite die charakteristisch für einzelne Abbauwege innerhalb eines Stoffwechselvorgangs sind.

Neben den gerichteten Metabolomics Untersuchungen ist es in dem zweiten Metabolomics Ansatz, dem Metabolic Fingerprinting, nicht beabsichtig jeden Metaboliten zu identifizieren, sondern es werden die Metabolitenmuster unterschiedlicher Tumorentiäten z.B. mit und ohne pharmakologischer Intervention verglichen. Wenn Unterschiede in den metabolischen Fingerabdrücken, unter Verwendung statistischer Methoden, festgestellt werden, werden die diskriminierenden Verbindungen der Strukturaufklärung unterworfen, um sie dann im biologischen Kontext zu interpretieren.

Die Erfahrungsdaten werden vorteilhafterweise vor oder nach einer Transplantation von Zellen oder sonstigen Bestandteilen eines NSB-Präparates bestimmt. Vorteilhafterweise können hierfür dem Spender, dem Empfänger und/oder dem NSB-Präparat Proben, wie beispielsweise Blutproben entnommen werden. Die Proben werden molekulardiagnostisch mittels insbesondere Metabolic Profiling analysiert. Diese Analyse umfasst biochemische, physikalische sowie chemische Untersuchungen. Die Daten der Analysen können einfach in das System eingegeben und von diesem gespeichert werden.

Vorteilhafterweise werden die Erfahrungsdaten, das heißt die für ein NSB-Präparat spezifischen und charakteristischen Werte, auf einem Speichermedium gespeichert. Das Speichermedium oder ein Datenspeicher dient im Sinne der Erfindung zur Speicherung von Daten beziehungsweise Informationen. Die Daten können vorteilhafterweise jederzeit um weitere ergänzt werden und liegen bevorzugt in digitaler Form vor. Es kann bevorzugt sein, dass das Speichermedium ein Massenspeicher mit bevorzugt magnetischer Aufzeichnungstechnik oder Halbleiterspeichertechnik ist. Ein Massenspeicher bezeichnet im Sinne der Erfindung ein Speichermedium, welches eine große Daten- oder Informationsmenge bevorzugt über einen längeren Zeitraum speichert. Vorteilhafterweise kann ein Massenspeicher mit magnetischer Auszeichnungstechnik verwendet werden, der binäre Daten auf die Oberfläche einer rotierenden, ferromagnetischen Scheibe schreibt. Halbleiterspeicher sind im Sinne der Erfindung Datenspeicher, die aus einem Halbleiter bestehen, in die mittels der Halbleitertechnologie integrierte Schaltkreise realisiert werden. Die Daten werden in Form von binären elektronischen Schaltzuständen in den integrierten Schaltungen gespeichert. Hierdurch ist eine dauerhafte und sichere Verwahrung der Daten möglich.

Die Anfragedaten werden ebenfalls mittels Verarbeitungseinheiten in das System eingegeben und auf einem Speichermedium gespeichert. Die Anfragedaten sind im Sinne der Erfindung Daten, die insbesondere den Empfänger der NSB-Präparate charakterisieren. Die Anfragedaten umfassen vorteilhafterweise ebenfalls molekulardiagnostische Daten, das heißt z.B. Informationen über Metabolite, die beispielsweise durch Metabolic Profiling generiert werden. Vergleichbare Daten können auf Ebene des Proteoms z.B. durch Phosphoproteomik oder auch auf Ebene des Transkriptoms, des Genoms oder des Epigenoms erhoben werden. Diese Daten können bereits vor Therapie des Patienten z.B. in einer persönlichen Datenbank, wie sie z.B. Microsoft mit HealthVault anbietet, vorliegen oder im Rahmen der Therapie von der betreffenden Klinik erhoben werden oder im Rahmen des Programms zur Entwicklung von Medikamenten, wobei vorzugsweise Daten vor und nach der Therapie des betreffenden Patienten erhoben werden, um endogene und exogene Faktoren besser bewerten zu können. Das System erlaubt es, diese Daten in geeigneter z.B. anonymisierter Form zu ordnen und hinsichtlich einer speziellen Fragestellung zusammenzufassen und dem Fragesteller z. B. im Zusammenhang von Entwicklungen von Medikamenten zur Verfügung zu stellen.

Die Anfragedaten umfassen vorteilhafterweise Genom-, Transkriptom-, Proteom-, Epigenom- und/oder Metabolomdaten und können beispielsweise von spezialisierten Einrichtungen oder Firmen (z. B. Genomsequenzierungseinrichtungen) bestimmt werden.

Metabolite, die beispielsweise durch das Metabolic Profiling bestimmt werden, stammen vorzugsweise aus dem Gewebe, den Schleimhäuten, der Lunge, der Leber, dem Blut, dem Darm, dem Kot, der Niere und/oder dem Urin. Metabolite bezeichnen bevorzugt aus dem Stoffwechsel (Metabolismus) resultierende oder im Stoffwechsel umgesetzte Produkte. Hierbei wird zwischen endogene und exogenen Metaboliten unterschieden. Endogene Metabolite werden durch den Organismus selbst synthetisiert (zum Beispiel Hormone) und im Falle von Mikroorganismen auch industriell genutzt. Exogene Metabolite werden durch einen Organismus erzeugt und durch einen anderen aus der Umwelt aufgenommen (zum Beispiel Vitamine). Die intrazelluläre Konzentration eines Metaboliten ergibt sich aus der Summe seiner positiven Bildungs- und negativen Umwandlungs- und Ausscheidungsraten. Bei Mutationen mit einem genetischen Defekt innerhalb eines Stoffwechselwegs reichern sich im allgemeinen die vor der Mutationsstelle gelegenen Metaboliten an. Als Primärmetabolite werden dabei diejenigen Zwischenprodukte entlang der Abbau- und Aufbauwege bezeichnet, die für die Zelle von zentraler Bedeutung sind. Davon unterschieden werden die sogenannten Sekundärmetabolite: Verbindungen, die zumeist auf Nebenwegen, oft unter Hunger- oder Mangelbedingungen oder nach Abschluss der aktiven Wachstumsphase der Zelle, gebildet werden und für die in vielen Fällen keine erkennbaren Funktionen für die Zelle bekannt sind (z. B. Wachse, Harze, Gerbstoffe, Farbstoffe, ätherische Öle, Antibiotika oder Alkaloide).

Die Anfragedaten können vorteilhafterweise direkt bei der Erstellung in das System eingegeben werden, wobei dieser Prozess bevorzugt automatisch abläuft. Hierbei können beispielsweise nach erfolgreicher Bestimmung der Metabolite, die Daten in das System übertragen werden. Das System bietet hierfür entsprechende Datenspeicher an, die unterschiedliche Datenformate verarbeiten können. Vorteilhafterweise werden unterschiedliche Datenformate auf ein einheitliches und vergleichbares Format gebracht. Dies stellt einen wesentlichen Vorteil gegenüber den im Stand der Technik beschriebenen Datenbanken oder Datenverwaltungsprogrammen dar, da erstmals ein einfacher und vor allem schneller Austausch von Daten möglich ist.

Es werden vorteilhafterweise Informationen über Patient und Präparat (z.B. HLA Werte oder Gewicht und Zellzahl) durch informationsverarbeitende Systeme miteinander in Korrelation gesetzt und zur Auswertung von Übereinstimmungen genutzt. Die Informationen über die verfügbaren Nabelschnurblutpräparate (NSBP) werden vorteilhafterweise dezentral durch die Blutbanken zur Verfügung gestellt und aktualisiert. Die Informationen über das verfügbare NSBP Inventar laufen beispielsweise in einem Repository (Datenbank) zusammen und werden dort zur Suche bereitgestellt.

Nachdem die Daten - die Erfahrungsdaten sowie die Anfragedaten - in das System eingegeben und gespeichert wurden, kann anhand einer Voreinstellung der Suchkriterien nach einem Match in dem System gesucht werden. Während der Recherche wird ein Vergleich der Erfahrungsdaten mit den Anfragedaten durchgeführt und eine automatische Auswertung der Recherche dem Suchenden angezeigt. Es kann jedoch auch bevorzugt werden, dass das System die Recherche in regelmäßigen oder unregelmäßigen Abständen automatisch durchführt und das Resultat der Recherche an den Suchenden (umfassend eine Klinik oder eine NSB-Bank) übermitteln. Vorteilhafterweise kann das Suchergebnis oder die Auswertung hierarchisch ausgegeben werden. Die Treffer, die eine komplette Übereinstimmung mit den Suchkriterien ergaben, können beispielsweise höher bewertet oder unterschiedlich gewichtet werden, als die bei denen keine komplette Übereinstimmung zutrifft. Hierdurch können die Ergebnisse übersichtlich angezeigt werden und der Suchende kann sich eher einen Überblick über das Ergebnis machen. Die Auswertung des Rechercheergebnisses erfolgt automatisch und kann von dem Suchenden manuell angepasst werde, indem die Suchkriterien unterschiedlich gewichtet werden.

Die Empfänger oder die für diese verantwortlichen Kliniken können die Kriterien, auf denen die Suche nach einem Match erfolgen soll, genau definieren. Zur Effizienzsteigerung und zur Fehlerminimierung können beispielsweise für die behandelnden Ärzte und Kliniken die Suchparameter, welche bei der Gewichtung und automatisierten Auswahl verwendet werden, zentral gespeichert werden. So können zum Beginn einer Suche die voreingestellten Suchparametersätze abgerufen werden und ggf. von einem Experten modifiziert werden (Expertenmodus). Die Recherche nach passenden NSBP läuft vorteilhafterweise automatisch ab, kann aber auch von einem Fachmann schrittweise ausgeführt bzw. überprüft werden.

Anhand der Auswertung der Recherche kann das NSB-Präparat bei der Nabelschnurblutbank oder Klinik bestellt werden. Vorteilhafterweise erfolgt die Bestellung über das Netzwerk und kann so auch über weite Strecken erfolgen, ohne das mit der entsprechenden Bank Kontakt aufgenommen werden muss. Hierfür sind die Verarbeitungseinheiten und/oder die Speichemedien mit dem Fachmann bekannten Datenübertragungseinheiten ausgestattet, die eine schnelle Datenübertragung ermöglichen. Beispiele hierfür sind DSL, ISDN oder sonstige Verbindungen die zur Kommunikation zwischen Verarbeitungseinheiten eingesetzt werden können. Zur Vorbereitung einer Bestellabwicklung kann ggf. eine Interaktion mit der Blutbank vorteilhaft sein, um weiterführende oder fehlende Untersuchungen zu veranlassen. Dies ist bisher ein manueller und zeitaufwändiger Schritt. Vorteilhafterweise unterstützt das System die Abläufe über einen automatisierten Workflow, das heißt ein Arbeitsablauf, der in einer vordefinierten Abfolge von Aktivitäten in einer Organisation abläuft. Der Workflow informiert stets über die anstehenden Aufträge und über den Arbeitsstand einzelner Aufträge, wodurch die Qualität der Resultate verbessert wird, sowie die Abläufe an sich effizienter und schneller ablaufen. Bei der Nachverfolgung der gelieferten und transplantierten Präparate ist das System in der Lage, die medizinisch und pharmakologisch notwendigen Informationen zusammenzutragen. Außerdem kann das System in einer bevorzugten Ausführungsform automatisiert Statistiken über die Abwicklungsgeschwindigkeit und die Geschwindigkeit der Blutbanken, sowie Erfolgsstatistiken in Abhängigkeit von Krankheitstypen und NSBP Parametern erstellen. So erhält der Benutzer des Systems, beispielweise der Koordinator eine übersichtliche Darstellung der Abwicklung und kann anhand dieser ggf. Arbeitsprozesse, beziehungsweise Bestellabwicklungen verbessern, da er nützliche Auswertungen über beispielsweise die Blutbank erhält.

Es war völlig überraschend, dass die potentiellen NSB-Präparate nach einem HLA-Match, einem Patientengewicht, einer Anzahl der kernhaltigen Zellen (TNC) und einer Anzahl der hämatopoetischen Zellen (CD34+) geordnet und ausgewählt werden können und zusätzlich vor und nach einer Transplantation und/oder einer Therapie von biologischen Zellen und/oder Geweben Metabolite, Genom-, Proteom-, Transkriptom- und/oder Epigenomdaten der Nabelschnurblut-Präparate, des Empfängers und/oder des Spenders bestimmt werden. Das System hat vorzugsweise Zugang zu den relevanten metabolischen Daten der Transplantate seien es Knochenmarkpräparate bzw. deren Spender oder seien es so genannte "Unit Reports" von Nabelschnurpräparaten. Diese metabolischen Daten können vor und nach der Transplantation mit den metabolischen Daten des Empfängers verglichen werden. Es hat sich überraschenderweise herausgestellt, dass wenn zusätzlich vor und nach der Transplantation Genom-, Proteom-, Transkriptom- und/oder Epigenomdaten von dem System erhoben werden, die Erfolgsaussichten für eine Transplantation maßgeblich erhöht werden können. Anhand der Daten kann der behandelnde Arzt oder die Klinik eine ausführliche Diagnose erstellen, die außerdem der NSB-Bank zur Verfügung gestellt werden kann. Diese kann einfach und schnell über das System auf die Daten zugreifen. Vorteilhafterweise bleibt die Vernetzung zwischen Entnahmezentrum, Lagerstätte, Klinik, Transplantationszentrum und/oder Forschungseinrichtung durch das System über einen längeren Zeitraum auch nach der Transplantation bestehen, wodurch eine konstante Beobachtung der Transplantationen sichergestellt ist. Das System kann bevorzugt die Verbindungen jederzeit unterbrechen oder erweitern und stellt diese automatisch her.

Die Genomdaten umfassen in einer bevorzugten Ausführungsform den einfachen (monoploiden oder haploiden) Satz der Erbsubstanz (d. h. doppelsträngige DNA), der in Eukaryoten im Wesentlichen in Form linearer Chromosomen vorliegt. Die Genomdaten werden vorteilhafterweise über eine Genomanalyse oder Genomsequenzierung oder "Genotyping" ermittelt. Hierbei wird die DNA eines Organismus, eines Organs, eines Gewebes oder Zellen isoliert und die Sequenzabfolge aller Basen-Paare der DNA bestimmt. Aus dieser Information kann eine Ursache-Wirkungsbeziehung zwischen Erbinformation (Sequenz) und der Eigenschaften des Organismus aufgefunden werden. Es kann jedoch auch bevorzugt sein, keine vollständigen Chromosomen, sondern nur Gene und/oder DNA von Eukaryoten zu analysieren. Das System kann Daten von Genom- oder Sequenzierungsanalysen verwenden, die vorteilhafterweise vor und nach der Transplantation entnommen und bevorzugt sequenziert werden. Die sequenzierten Proben können von dem System dazu benutzt werden, Mutationen, Genaberrationen oder Chromosomenaberrationen festzustellen, die ggf. im Zusammenhang mit der Transplantation stehen. Außerdem können so schnell und einfach Risikogruppen durch das System identifiziert werden, die beispielsweise genetische Mutationen aufweisen, die eine Transplantation erschweren und eine sorgfältige Behandlung des Empfängers nötig machen. Diese Risikogruppen konnten überraschenderweise nur identifiziert werden, weil auch nach der Transplantation Proben entnommen und mithilfe des Systems analysiert wurden und die Analysen Genom-, Proteom-, Epigenom-, Transkriptom- und/oder Metabolomanalysen umfassen. Nur durch diese hochauflösenden Untersuchungen können Veränderungen detektiert werden, die mit Standardmethoden nicht feststellbar sind. Die generierten Daten können von dem System dazu verwendet werden, Korrelationen zwischen genetischen Aberrationen und dem Erfolg einer Transplantation (oder Abstoßungsreaktion) zu errechnen. Eine Abstoßungsreaktion des Transplantates kann durch eine Früherkennung, d. h. durch eine hochauflösende Genomanalyse, die vor der Transplantation durchgeführt wird, vermieden werden.

Weiterhin können vorteilhafterweise Transkriptomdaten in das System eingegeben und gespeichert werden. Das Transkriptom bezeichnet bevorzugt die Gesamtheit aller von einem Organismus unter bestimmten Umweltbedingungen synthetisierten Gen-Transkripte (mRNA). Vor allem die Entwicklungen der DNA-Chip-Technologie, die bei hoher Sensitivität einen hohen Probendurchsatz mit geringem Ausgangsmaterial erlauben, machen umfangreiche Transkript-Analysen möglich. Ein solcher DNA-Chip, der das gesamte Genom eines Organismus abdeckt, wird mit der aus Zellen oder Geweben isolierten mRNA (oder der daraus umgeschriebenen cDNA) hybridisiert, um die transkribierten Gene zu identifizieren. Hierdurch kann die Expression bestimmter Gene schnell und effizient determiniert werden. Die bestimmte Expressionsrate eines Gens kann beispielweise Auskunft über die Aktivierung oder Deaktivierung bestimmter Signaltransduktionswege geben, die wiederum für die Diagnose oder Therapie von Krankheiten genutzt werden kann. Die Transkriptomdaten werden insbesondere vor und nach einer Transplantation erhoben und geben so eine umfangreiche Auskunft über die Expression bestimmter Gene. Diese Informationen können für die Beurteilung der Transplantation herangezogen werden und ebenfalls für die Früherkennung von Abstoßungsreaktionen genutzt werden.

Die Proteomdaten, die ebenfalls als Anfragedaten in das System eingegeben und gespeichert werden können, umfassen insbesondere die Gesamtheit aller Proteine in einem Lebewesen, einem Gewebe, einer Zelle oder einem Zellkompartiment, vorteilhafterweise unter exakt definierten Bedingungen und zu einem bestimmten Zeitpunkt. Die Analyse und Bestimmung des Proteoms erfolgt mittels Proteomik. Proteomik umfasst die Bestimmung der Gesamtheit der exprimierten und durch Posttranslation modifizierten Proteine unter definierten Bedingungen mit dem Ziel, Aussagen über den Zustand der Zelle zu erhalten sowie durch Vergleich des Proteinmusters einer Zelle oder eines Organismus unter verschiedenen Bedingungen komplexe physiologische Vorgänge zu entschlüsseln. Das System ist insbesondere für die Eingabe und Speicherung von Daten aus der Phosphoproteomik geeignet. Zellwachstum und -differenzierung werden über ein komplexes Netzwerk von zellulären Signalwegen gesteuert. Proteinkinasen nehmen hierbei eine Schlüsselrolle ein. Sie übertragen Phosphatgruppen auf andere Proteine und regulieren somit deren Enzymaktivität. Proteinphosphorylierungen treten bei schätzungsweise 30% aller Proteine auf. Sie stellen den wichtigsten Regulationsmechanismus in der Kontrolle von zellulären Signalwegen dar. In Krebszellen weisen bestimmte Proteine jedoch einen stark erhöhten Phosphorylierungsgrad auf. Diese Überaktivierung von Signalwegen ist charakteristisch für Krebserkrankungen. Außerdem hat sich herausgestellt, dass ebenfalls im Rahmen einer Transplantation, Änderungen der Proteinaktivitäten erfolgen, die im Wesentlichen auf Phosphorylierungen zurückzuführen sind. Die Phosphoproteomik zielt darauf ab, alle zellulären Proteinphosphorylierungen in Zell- und Gewebeproben quantitativ zu erfassen. Dabei ist der Einsatz modernster massenspektrometrischer Verfahren unumgänglich. Um quantitative Aussagen über den Phosphorylierungsgrad treffen zu können, können die Proteine der zu vergleichenden zellulären Zustände (z. B. mit und ohne Wirkstoff) mit massenspektrometrisch unterscheidbaren Isotopen markiert werden. Vor der eigentlichen massenspektrometrischen Analyse können die phosphorylierten Proteine zunächst enzymatisch in Peptidfragmente zerlegt und mithilfe spezieller chromatographischer Verfahren angereichert werden. Im Massenspektrometer können anschließend die genauen Massen der Phosphopeptide und ihre durch Kollision mit Gasmolekülen entstandenen charakteristischen Fragmentmassen bestimmt werden. Mithilfe bioinformatischer Methoden lassen sich aus den Messergebnissen schließlich die gemessenen Phophopeptide auf Proteinsequenzen abbilden und relative quantitative Veränderungen zwischen den analysierten Proben ableiten. Das System ist vorteilhafterweise in der Lage, Proben vor und nach der Transplantation zu entnehmen, beziehungsweise z. B. eine Klinik zur Probenentnahme zu beauftragen und die Proben insbesondere mithilfe der Phosphoproteomik zu analysieren. Die Analyse gibt eine detaillierte Aussage über beispielsweise den Phosphorylierungsgrad definierter Proteine wieder. Dies kann dazu genutzt werden, bevorzugt im Zusammenhang mit einer Genom- und/oder Transkriptomanalyse, die Aktivierung einzelner Proteine oder Signalkaskaden zu bestimmen. Anhand der Daten kann das System überraschenderweise Abstoßungsreaktionen extrem frühzeitig erkennen und ggf. eine Behandlung initiieren. Es war völlig überraschend, dass das System durch die Entnahme von Proben nach einer Transplantation und die Genom-, Transkriptom-, Epigenom- und/oder Proteomanalyse der Proben, Abstoßungsreaktionen früher als bei im Stand der Technik offenbarten Methoden erkennen kann. Die generierten Daten können die Effizienz der Transplantation einfach und schnell bestimmen. Die Ergebnisse geben so eine detaillierte Auskunft über die Transplantation und können ebenfalls für eine Transplantationsoptimierung herangezogen werden. Die Daten sind, bedingt durch die Vernetzung der Beteiligten durch das System, für jeden verfügbar. Hierdurch kann die Anzahl der Untersuchungen erheblich reduziert werden.

Weiterhin können Metabolomdaten in das System eingepflegt werden. Hierbei handelt es sich bevorzugt um die Gesamtheit der zellulären Metabolite, d. h. der in den Reaktionen des Stoffwechsels erzeugten (zumeist niedermolekularen) chemischen Substanzen. Vorteilhafterweise können ebenfalls Durchflussraten oder Umsatzraten bestimmter Metabolite, der Metabolit-Spiegel und Enzymaktivitäten einzelner Stoffwechselwege und/oder Kompartimentierung der verschiedenen Stoffwechselwege innerhalb eines Organismus, eines Organs, eines Gewebes oder einer Zelle bestimmt werden. Vorteilhafterweise ist es möglich Daten aus Metabolom-Analysen direkt zu verwenden. Bei den Metabolom-Analysen (Metabolomiks) werden kleine chemische oder biologische Moleküle - Metabolite - detektiert und quantifiziert. Hierfür können unterschiedliche Proben herangezogen werden, umfassend Blutplasma, Blutserum, Urin, CSF, Zellextrakte oder Gewebe. Die Proben werden anhand von state-of-the-art Analysemethoden, wie beispielsweise Spektralanalyse, Massenspektroskopie und/oder chromatographischen Methoden charakterisiert und die biologische und/oder biochemische Interaktion mit anderen Molekülen modelliert. Hierdurch kann das Molekül in einen oder mehrere Signalkaskaden oder Stoffwechselwege eingeordnet werden. Außerdem können die Metabolite dazu verwendet werden, einen Effekt von Arzneimitteln auf Stoffwechselvorgänge zu bestimmen. Vorteilhafterweise werden Proben vor und nach der Transplantation an beispielsweise Firmen geschickt, die eine Analyse der Metabolite durchführen können. Hierdurch ist eine Beobachtung der Transplantationseffekte über einen langen Zeitraum möglich, wobei die generierten Daten von dem System ebenfalls für Analysen und Statistiken verwendet werden können, die die Transplantationsstrateglen oder -effizienz verbessern können.

Vorteilhafterweise können ebenfalls Epigenomdaten in das erfindungsgemäße System eingegeben und von diesem analysiert und verwertet werden. Epigenetische Änderungen beschreiben Änderungen in der Aktivität von Genen, die nicht die Sequenzebene (wie Mutationen etc.) betreffen. Die Hauptmechanismen einer epigenetischen Änderung betreffen das Chromatin, das in spezifischer Weise umgebaut und modelliert werden kann. Die Änderungen des Chromatins kann beispielsweise durch Prozesse umfassend Methylierung oder Acetylierung einzelner Chromatin-Elemente erfolgen. DNA-Methylierung ist ein strikt kontrollierter biologischer Prozess, der der natürlichen Regulation von Genen und der Stabilität des Genoms dient. Cytosin kann durch anhängen einer chemischen Methlygruppe modifiziert werden. DNA-Methylierung in genregulatorischen Regionen (d.h. Genpromotoren) schaltet gewöhnlich die betroffenen Gene stumm in dem sie entweder direkt die Bindung von Transkriptionsfaktoren verhindert oder eine Kondensation des Chromatins ermöglicht. Weil verschiedene Zellen verschiedene Gene abschalten, hat jeder Zelltyp seinen spezifischen "DNA-Methylierungs-Fingerabdruck". Dieser "Fingerabdruck" ist insbesondere beim Altem und bei Krankheiten spezifisch verändert und daher eine Quelle für Biomarker für die organspezifische Diagnose und Klassifikation von Krankheiten. Es können beispielsweise DNA-Methylierungsmuster dazu benutzt werden, DNA die in Körperflüssigkeiten umfassend Blut oder Urin abgesondert wurde, hochempfindlich nachzuweisen und anhand der Methylierungsmuster einen Rückschluss auf die Genaktivität zu machen. Vorteilhafterweise können die Methylierungsmuster mithilfe von minimalinvasiven Methoden bestimmt werden, wodurch beispielsweise frühzeitig eine Abstoßungsreaktion nachweisbar ist. Weiterhin kann DNA-Methylierung einfach quantifiziert werden, da die unmethylierte Kopie des gleichen Gens in der Probe als interne Referenz genutzt werden kann. Die Methylierung von Zell- oder Gewebeproben kann einfach in High-Throughput-Analysen, wie beispielsweise Microarray Technologie oder PCR oder Sequenzierungstechnologien, bestimmt werden. Es hat sich überraschenderweise gezeigt, dass durch die Probenentnahme nach einer Transplantation und der Epigenomanalyse, Gene oder Gencluster identifiziert werden können und das System eine Berechnung über die Wahrscheinlichkeit einer erhöhten Abstoßungsreaktion von Transplantaten durchführen kann. Nur durch eine Probenentnahme vor und nach der Transplantation können diese Risikogruppen oder -patienten durch das System einfach und schnell identifiziert werden. Diese Patienten können dann umfangreich behandelt werden, wodurch die Abstoßungsreaktion erheblich reduziert und ein erfolgreicher Transplantationsverlauf sichergestellt werden kann.

Das Epigenom beschreibt im Sinne der Erfindung die Gesamtheit der epigenetischen Merkmale, d. h. alle meiotisch und mitotisch vererbbaren Veränderungen in der Genexpression, die nicht in der DNA-Sequenz selbst kodiert sind.

Damit kann das System automatisch auch nach Jahren die metabolischen Daten des Patienten für das Register oder die Nabelschnurblutbank abrufen. Diese metabolischen Daten können vom System auch anonymisiert und für statistische Auswertungen bereit gestellt werden.

Moderne molekulare Diagnose Methoden wie zum Beispiel Metabolic Profiling von BioMarkern können ohne solch ein automatisiertes System nicht validiert und z.B. für die Entwicklung neuer Arzneimittel eingesetzt werden. Darüber hinaus können metabolischen Daten aus beispielsweise dem Metabolic Profiling später den Verlauf der Transplantation und die Genesung des Patienten wesentlich genauer diagnostizieren. Die standardmäßige Einbeziehung von metabolischen Daten in alle Transplantationen und die mögliche statistische Auswertung bedeutet für die regenerative Medizin einen Zeitsprung. Das beschriebene System erlaubt dies erstmalig, da es automatisiert ist und über die entsprechenden Vernetzungen z.B. des Anbieters von Metabolic Profiling mit Registern/Banken einerseits und andererseits mit den Transplantationszentren und Kliniken verfügt.

Es war völlig überraschend, dass die Entnahme von Proben aus insbesondere dem Gewebe, den Schleimhäuten, der Lunge, der Leber, dem Blut, dem Darm, dem Kot, der Niere und/oder dem Urin und die Bestimmung der Metabolite und der Genom-, Proteom-, Transkriptom- und/oder Epigenomanalyse vor und nach der Transplantation, die Abstoßungsreaktionen eines Transplantates wesentlich verringert werden kann. Die Bestimmung definierter Metabolite mithilfe des Systems ermöglicht erstmalig die genaue Analyse der Beeinträchtigung von Stoffwechselwegen durch eine Transplantation. Zusätzlich können durch Genom-, Proteom-, Transkriptom- und/oder Epigenomanalysen molekulargenetische oder biochemische Änderungen festgestellt werden. Hieraus können Informationen zur weiteren Therapie der Patienten abgeleitet werden. Die gesammelten Daten können weiterhin für Arzneimittelstudien genutzt werden. Vorteilhafterweise kann die Analyse der Proben und die Zeitpunkte zur Probeentnahme automatisch über das erfindungsgemäße System bestimmt und reguliert werden.

Es war durch den Stand der Technik nicht absehbar, dass insbesondere die Bestimmung von metabolischen Daten vor und nach einer Transplantation die Transplantationserfolge maßgeblich verbessern. Insbesondere anhand der Probenentnahme nach der Transplantation können Abstoßungsreaktionen des Transplantates überraschend durch das System frühzeitig erkannt werden, da bestimmte Metabolite oder Biomarker frühzeitig gebildet werden und diese mithilfe von Standardanalysen nicht feststellbar sind. Dies stellt einen erheblichen Fortschritt dar und löst ein lang bestehendes Problem. Es steht dem Fachmann nun ein System zur Verfügung, durch welches beispielsweise Ergebnisse eines Metabolic Profilings direkt genutzt und mit bestehenden Datensätzen verglichen werden können. Dies stellt für die Transplantationsmedizin erhebliche Vorteile dar, da die Kosten für aufwendige diagnostische Untersuchen wegfallen und hierfür auf bereits vorhandene metabolische Daten zurückgegriffen werden können. Hier stehen den Ärzten und Kliniken erheblichen Datenmengen zur Verfügung, die ihnen eine umfangreiche Beurteilung und Diagnose ermöglichen. Hierdurch kann die Transplantation nicht nur sicherer gemacht werden, sondern auch Vorsorge betrieben werden, wodurch wiederum staatliche und private Krankenkassen Geld sparen können, da teure und umfangreiche Vorsorgeuntersuchungen gegen kostengünstige Analysemethoden, wie beispielsweise das Metabolic Profiling ausgetauscht werden.

Die Erfindung ist neu und erfinderisch und beschreibt ein automatisiertes System zur Verwaltung und Bereitstellung individueller oder gepoolter und auch anonymisierter Patientendaten auf Grundlage der molekularen Diagnostik auf Ebene der Genoms, des Transkriptoms, des Proteoms, des Epigenoms oder des Metaboloms vor und nach Transplantation oder Therapie von chemischen, biochemischen, biologischen oder Zellprodukten als individuelle oder gepoolte Follow-up Datensammlung auch anonymisiert oder allgemeine oder editierte Datensammlung für insbesondere die Arzneimittel Entwicklung. Es können so vor und nach einer Transplantation Proben entnommen und analysiert werden, die insbesondere für die Follow-Up Benhandlung der Empfänger wichtig ist. Dem Fachmann wird die Erfindung auch nicht durch den Stand der Technik nahegelegt, sondern war völlig überraschend für ihn. Im Stand der Technik ist nichts dergleichen beschrieben. Der Stand der Technik offenbart im Wesentlichen nur die Standardanalyse von Proben nach einer Transplantation (z. B. Blutanalysen), wobei keine Genom-, Proteom-, Transkriptom-, Epigenom- und/oder Metabolomanalysen durchgeführt werden. Nur durch die Erhebung dieser Daten nach einer Transplantation und die Analyse durch das erfindungsgemäße System, können die realen Wirkungen der Transplantation auf den Empfänger bestimmt werden. So hat sich überraschenderweise gezeigt, dass durch die Analysen mithilfe des Systems Risikogruppen identifiziert werden können, die eine hohe Wahrscheinlichkeit für eine Abstoßungsreaktion aufweisen. Die gewonnenen Daten können durch das System analysiert werden und zur Verbesserung der Transplantationsstrategie oder für die Entwicklung von Behandlungsmethoden genutzt werden. Die Verwendung dieser Daten eröffnet dem Fachmann ein völlig neues technisches Gebiet, da die Daten sehr detailliert sind und nicht nur transplantationsspezifische Faktoren berücksichtigen. Außerdem werden die Daten automatisch von dem System erstellt, angefordert und verwaltet und stehen den vernetzten Beteiligten zur Verfügung. Bevorzugt ist, dass bei der Eingabe der Daten, d. h. der Erfahrungsdaten insbesondere alle in dem System registrierten NSB-Präparate, die weltweit in verschiedenen NSB-Banken oder Entnahmezentren lagern, mit einem vorteilhafterweise einheitlichen Datensatz (Unit Report) als Parameter erfasst werden. Die Parameter umfassen:

- Name und Kennung der lagernden NSB-Bank

- Status der lagernden NSB-Bank hinsichtlich internationaler Zertifizierungen (z.B. Fact)

- Abwicklungszuverlässigkeit der NSB-Bank nach Klassifizierung

- Ansprechpartner an der betreffenden Bank mit Kontaktdaten

- Identifikationsnummer des Präparates

- Anamnese der Mutter, des Kindes und der Familie gemäß Anamnesebogen der Geburtsklinik

- Ethnie der Mutter, des Vaters und/oder des Kindes

- Geschlecht des Kindes

- Einlagerungszeitpunkt des Präparates

- Informationen zur Aufarbeitung des Präparates

- Blutgruppe des Präparates

- HLA-Typus des Präparates

- Zellzahl (TNC) des Präparates

- Zellzahl (CD34+) des Präparates

- Virusstatus des Präparates

- Allele Ausprägungen des Präparates,

- Molekulare Diagnosen und Analysen des Genoms, Transkriptoms, Proteoms, Epigenoms und/oder Metaboloms,

wobei der Datensatz auf einem Speichermedium und/oder Verarbeitungseinheit gespeichert werden. Die kombinierten Parameter werden vorteilhafterweise in das System eingepflegt und ermöglichen überraschenderweise die eindeutige Bezeichnung eines Nabelschnurblutpräparates (NSBP), da aufgrund der eingepflegten Daten, beziehungsweise der Kombination der Parameter, jedes Präparat durch seine spezifischen Eigenschaften oder Parameter definiert wird. Dies wird vorteilhafterweise durch die kombinierte Erfassung der Parameter erreicht. Im Sinne der Erfindung beschreibt ein Parameter eine Kenngröße, das heißt eine charakterisierende Eigenschaft, die als Daten in das System eingefügt werden. Vorteilhafterweise umfassen diese operationelle Informationen (Attribute) von Patienten, Kliniken, Ärzte, Spender, Blutbanken, NSB-Präparate (Laborwerte, physikalische und informationelle Eigenschaften), Bestell- und Abwicklungs-Informationen und steuernde Informationen umfassend Such- / Ausschlusskriterien, Schwellwerte, Gewichtungsfaktoren. Insbesondere durch molekulare Diagnosen und Analysen des Genoms, Transkriptoms, Proteoms, Epigenoms und/oder Metaboloms können die NSB-Präparate oder sonstige Zell- oder Gewebepräparate, die sich zur Transplantation eignen, detailliert charakterisiert werden. Die Analysen geben beispielsweise nicht nur Auskunft über Stoffwechselaktivitäten, sondern auch über potentielle Mutationen. Hierdurch können NSB-Präparate hierarchisch geordnet werden und machen so die Auswahl eines passenden Präparates einfacher und schneller. Es war völlig überraschend, dass die Kombination der genannten Parameter zu einer bevorzugten guten Lösung des erfindungsgemäßen Problems führt.

Hierbei werden Parameter erfasst, die zur Kennzeichnung der Präparate verwendet werden. Besonders die vorteilhafte Kombination der Parameter ist nicht im Stand der Technik beschrieben und ermöglicht eine eindeutige Zuordnung und Erfassung eines Präparates. So kann eine Datenbank, im Sinne der Erfindung eine NSBP Datenbank angelegt werden, in die die Parameter gespeichert werden.

Die Eingabe der Parameter kann beispielsweise dezentral durch die NSB-Bank erfolgen, wobei auch die Datenbank von dieser gepflegt bzw. aktualisiert werden kann. Jedoch können die Daten ebenfalls von externen Dienstleistern zur Verfügung gestellt werden. Dies ist von Vorteil, da die Klinik oder die NSB-Bank teure und aufwendige Analysen nicht selbst durchführen muss und somit Ressourcen eingespart werden. Die Dienstleister können beispielsweise vor und nach einer Transplantation von dem System Proben automatisch zur Verfügung gestellt bekommen, wobei die generierten Daten in das System eingepflegt werden und für die Beteiligten zugänglich sind.

Es werden neben den Informationen der NSB-Bank, wie beispielsweise Name und Kennung der NSB-Bank auch der Status der Bank hinsichtlich internationaler Zertifikationen (z.B. Fact - "Foundation for the Accreditation of Cellular Therapy") gespeichert, wodurch sichergestellt werden kann, dass definierte Normen bezüglich der Qualität der Präparate eingehalten werden. Auch ein Ansprechpartner an der betreffenden Bank mit Kontaktdaten kann vorteilhafterweise eingetragen werden. Hierbei umfasst ein Ansprechpartner beispielsweise einen behandelnden Arzt oder einen Koordinator, der an der Bank für die Pflege der Datenbank zuständig ist. Weiterhin wird bevorzugt eine systemeinheitliche Identifikationsnummer (ID) vergeben, die eine eindeutige Zuordnung ermöglicht. Außerdem ist so die Suche nach Präparaten von der NSB-Bank, sowohl übergreifend gegeben. Zusätzlich werden Abwicklungszuverlässigkeitsinformationen für jede NSB-Bank durch das System automatisiert erhoben und bei der Suche berücksichtigt. Des weiteren werden Daten zu der Anamnese der Mutter, des Kindes und der Familie gemäß eines Anamnesebogens der Geburtsklinik in die Datenbank mit aufgenommen. Hierdurch ist vorteilhafterweise eine Beurteilung der Präparate bezüglich bestimmter Krankheiten, beispielsweise Erbkrankheiten möglich. Die Ethnie der Mutter, des Vaters und/oder des Kindes ist als Informationen vorteilhaft, da bestimmte genetische Variationen mit dem ethnischen Hintergrund assoziiert sein können und somit ggf. eine Transplantation verkomplizieren können. Vorteilhafterweise werden weiterhin Parameter wie Blutgruppe, HLA-Typus, Zellzahl (TNC - "total nuclear cells" und CD34+), Virusstatus und allele Ausprägung der Präparate in die Datenbank eingepflegt. Diese umfangreichen Information ermöglichen eine Charakterisierung und Identifikation der Präparate und dementsprechend eine optimale Zuordnung eines Empfängers.

Vorteilhafterweise beinhaltet der Datensatz jeden Präparates Informationen darüber, ob das Präparat in Segmenten (wenn ja, wie viele) und mit Fragmenten (wenn ja, wie viele) und mit DNA-Samples (wenn ja, wie viele) eingefroren wurde. Fragmente, Segmente und Samples dienen der späteren näheren Bestimmung des Präparates hinsichtlich eines bestimmten Patienten und zur Überprüfung zentraler Daten vor der Transplantation. Das System informiert, wie viele der Segmente, Fragmente und DNA-Samples aktuell bei der Anfrage noch vorhanden sind, bzw. welche weiteren Tests wie z.B. CT (Confirmatory Typing) HR (High-Resolution HLA-Typing), CA (Colony Assays) oder Genotyping bereits durchgeführt wurden bzw. welches die Ergebnisse dieser Test waren. Darüber hinaus wird der Status eines Präparates festgehalten, d.h. ob und seit wann das Präparat möglicherweise von einer Klinik reserviert ist.

Im Sinne der Erfindung kann die Datenbank, umfassend die Daten oder Parameter auch als ein Data Warehouse, d.h. eine zentrale Datensammlung, deren Inhalt sich aus Daten unterschiedlicher Quellen zusammensetzt, bezeichnet werden. Dieses verwaltet nicht nur alle Daten der einzelnen Präparate in den verschiedenen NSB-Banken, sondern es gleicht auch jedes Präparate, das eingestellt wird, dynamisch gegen alle anderen Präparate in den verschiedenen NSB-Banken ab, so dass automatisch mit Registrierung eines jeden Präparates dokumentiert wird, welche Präparate untereinander für eine möglichen späteren Doppel- oder Mehrfachtransplantation (MultiCord) eingesetzt werden können.

Das erste Ordnungskriterium für diesen Multi-Cord-Abgleich zwischen den registrierten Präparaten ist der HLA-Match, wobei es auch bevorzugt sein kann, dass das erste Ordnungskriterium die Blutgruppe oder die TNC-Zellzahl ist. Es kann ebenfalls bevorzugt sein, dass die Ordnungskriterien anhand von Parametern der molekularen Diagnostik erstellt werden. Hierbei sind insbesondere Biomarker, die für bestimmte Krankheiten spezifisch sind, vorteilhaft. Anhand dieser Kriterien, können bestimmte NSB-Präparate schnell und effizient gefunden werden. Weiterhin kann es bevorzugt sein, dass die Ordnungskriterien auf Daten des Genoms, Proteoms, Transkriptoms, Epigenoms und/oder des Metaboloms basieren. Es hat sich überraschenderweise herausgestellt, dass durch die Analyse des Genoms, Transkriptoms, Proteoms, Metaboloms und/oder des Epigenoms mittels des Systems, Risikopatienten und inkompatible NSB-Präparate schnell identifiziert werden. Das heißt, die NSB-Präparate werden insbesondere vor und nach einer Transplantation mithilfe des Systems analysiert, wodurch eine Korrelation zwischen charakteristischen Merkmalen der NSB-Präparate, des Empfängers und/oder des Spenders und einer Abstoßung des Transplantates bestimmbar ist. Die Auswertung dieser Daten durch das System kann bestimmte Merkmale identifizieren, die das Risiko für eine Abstoßung erhöhen können. Wenn dieses Risiko früh, d. h. vor der Transplantation erkannt wird, können diese inkompatiblen Präparate bei einer Auswahl vermieden werden. Falls keine alternativen Präparate zur Verfügung stehen, kann eine früh eingesetzte Therapie die Abstoßungsreaktion reduzieren oder sogar vollständig unterdrücken. Das System ermöglicht überraschenderweise durch die Ordnungskriterien, insbesondere durch die Genom-, Proteom-, Metabolom-, Epigenom und/oder Transkriptomanalyse, dass insbesondere nur vollständig kompatible NSB-Präparate für eine Transplantation verwendet werden.

In einer bevorzugten Ausführungsform besteht in mindestens vier von sechs HLA-Merkmalen bevorzugt Übereinstimmung. In der Reihenfolge der Eignung als Multi-Cord stehen diejenigen Präparate ganz oben, die die meisten HLA-Matches haben. Präparate, die z.B. einen negativen Virusstatus haben, das heißt einen bestimmten Virus nachweisbar nicht aufweisen, werden nicht berücksichtigt. Im Sinne der Erfindung beschreibt eine Ordnung eine definierte Reihenfolge von Elementen. Die Ordnung der Elemente kann auf deren Eigenschaften, beispielsweise die Parameter joder Attribute (beispielsweise NSB Präparate) bezogen sein. Im Sinne der Erfindung beschreiben die Ordnungskriterien, wie die Ordnung zu Stande kommt (beispielsweise alle NSB-Präparate gemäß Ihrer TNC Größe vom größten zum kleinsten Präparat aufreihen). Vorteilhafterweise können auf eine Ordnung Filterkriterien angewendet werden, das heißt es können beispielsweise nur Präparate für eine Suche berücksichtigt werden, die eine definierte TNC Größe aufweisen. Besonders vorteilhaft ist, dass bei größeren Datenmengen diese Ordnungen als Index verwendet werden kann, um beispielsweise effiziente Suchen (auch als Kombination über mehrere Kriterien hinweg) durchzuführen.

Das zweite Ordnungskriterium ist die Blutgruppen Gleichheit bzw. Verträglichkeit. Präparate mit Blutgruppen Gleichheit stehen wieder ganz oben, diejenigen mit Verträglichkeit folgen und Blutgruppen die sich ausschließen führen zur Nichteignung als MultiCord hinsichtlich bestimmter anderer Präparate.

Zellzahl (TNC und CD34+), ethnische Zugehörigkeit und Allele-Ausprägungen werden als Informationen oder Merkmale der Präparate mitgeführt und dienen zur Bestimmung der weiteren Reihenfolge, das heißt ob die Präparate geeignet wären und ob es vorteilhaft wäre, ein weiteres Merkmal der Präparate zu überprüfen. Präparate mit hoher TNC-Zellzahl und hoher CD34+ Zellzahl stehen wieder weiter oben. Gleiches gilt für identische ethnische Herkunft und verträgliche Allele Ausprägungen. Damit sind im Datenbestand des Systems, das heißt der in der Datenbank vorliegenden Daten, bereits vor Anfrage durch eine Klinik für einen individuellen Patienten, mögliche Paarungen bzw. Gruppierungen zueinander passender Präparate identifiziert und in Rangfolge gesetzt.

Bevorzugt ist, dass die anfragende Klinik eine Patientenrecherche durchführt, wobei die Ermittlung der zum Patienten kompatiblen Präparate entsprechend folgender Ordnungs- und/oder Ausschlusskriterien umfasst:

- Name und Kennung der Klinik bzw. des Transplantcenters

- Name des Koordinators und des behandelnden Arztes mit Kontaktdaten

- Status der Klinik hinsichtlich internationaler Zertifizierungen (z.B. Fact)

- Durchschnittliche Anzahl an NSB-Transplantationen an der anfragenden Klinik in den letzten drei Jahren

- Name des Patienten, Versicherungsnummer und weitere Abrechnungsdaten

- Anamnese des Patienten

- Indikation und Therapievorschlag des behandelnden Arztes

- Dringlichkeit nach definierter Klassifizierung

- HLA-Typus des Patienten

- Blutgruppe des Patienten

- Gewicht des Patienten

- Ethnie des Patienten

- Geschlecht des Patienten

- Alter des Patienten

- Bekannte Allele Ausprägungen des Patienten und/oder Daten einer DNA-Typisierung

- Erst- oder Wiederholungsbehandlung,

wobei die Ordnungs- und/oder Ausschlusskriterien auf einem Speichermedium und/oder Verarbeitungseinheit gespeichert werden.

Völlig überraschend ist in diesem Zusammenhang die Möglichkeit, dass genetische Abweichungen oder Mutationen und unterschiedliche Muster aus molekularen Diagnosen aus dem Genom, dem Transkriptom, dem Proteom, dem Epigenom und dem Metabolom als Ordnungs- bzw. Ausschlusskriterien mitgeführt werden, die auch nachfolgend erhoben werden. Das heißt, es kann auch vorteilhaft sein, die Präparate nach Eigenschaften des Genoms, Transkriptoms, Proteoms, Epigenoms und /oder Metaboloms zu ordnen. Diese Eigenschaften umfassen beispielsweise Mutationen, Expression bestimmter Gene, Anwesenheit/Abwesenheit von bestimmten Proteinen, Methylierung bestimmter Gene oder Chromatinabschnitte und/oder die Umsetzung bestimmter Stoffwechselprodukte. Durch die Erhebung dieser Daten können die Präparate eindeutig charakterisiert werden und vorteilhafterweise mit den Daten eines Empfängers mittels des Systems verglichen werden. Der Vergleich kann beispielsweise frühzeitig eine Inkompatibilität aufzeigen, die zu einem erhöhten Abstoßungsreaktion des Transplantates führen könnte. Außerdem können durch die hochauflösenden Analysen Defekte der NSB-Präparate entdeckt werden, die durch Standardmethoden nicht detektierbar sind. Die Daten sind von allen Beteiligten, die mit dem System verbunden sind, einsehbar, wodurch wiederum Mehrfachuntersuchungen vermieden und Kosten reduziert werden können.

Durch die vorteilhafte Kombination der Ordnungs- und/oder Ausschlusskriterien, die synergistisch zusammenwirken, kann ein Patient eindeutig charakterisiert werden, wobei vorteilhafterweise die Daten eines Patienten mit gespeicherten Daten eines Präparates in einer NSB-Bank verglichen werden. Der synergistische Effekt besteht darin, dass die erfindungsgemäße Kombination der Ordnungs- und/oder Ausschlusskriterien, beispielsweise von Genom- und Metabolomanalysen, eine überraschend genaue Charakterisierung der Präparate ermöglicht. Es hat sich überraschenderweise gezeigt, dass das System schnell passende Präparate findet, da eine größere Anzahl von charakteristischen Merkmalen zur Verfügung steht und keine weiteren Untersuchungen bezüglich der Kompatibilität durchgeführt werden müssen. Dies führt zu einer Reduzierung des Arbeitsaufwandes und der Kosten. Außerdem ist es für erfolgreiche Transplantationen die Geschwindigkeit, in der ein passendes Präparat gefunden wird, von enormer Wichtigkeit. Vorteilhafterweise werden Patient und Präparat mittels den gleichen Kriterien charakterisiert, wodurch ein direkter Vergleich möglich ist. Hierbei können vorteilhafterweise die Eigenschaften der Präparate mit denen eines Patienten über beispielsweise eine Verträglichkeitsmatrix in mehreren Stufen und verschiedenen Ordnungskriterien verglichen werden. Die Verträglichkeitsmatrix erlaubt einen direkten und einfachen Vergleich der Eigenschaften der Präparate mit denen des Patienten und gibt Auskunft, ob das Präparat verträglich für den Patienten ist. Vorteilhafterweise werden dem behandelnden Arzt mehrere Ergebnisse, das heißt Präparate präsentiert, die für einen Patienten optimal wären. Des Weiteren können vorteilhafterweise Präparate für SingleCords oder MultiCords hinsichtlich der Transplantation in einen bestimmten Patienten Vorgeschlagen werden. Die finale Entscheidung, welches Präparat oder welche Präparate zur Anwendung kommen, kann vorteilhafterweise durch den behandelnden Arzt getroffen werden.

Im Sinne der Erfindung beschreibt das Kriterium Indikation und Therapievorschlag des behandelnden Arztes, die Diagnose, Analyse und Indikation der Krankheit an der der Patient leidet (beispielsweise Akute Myeloische Leukämie (AML) oder Ischämischer Schlaganfall) und für die der behandelnde Arzt eine bestimmte Behandlung (Therapie) vorschlägt. Der Therapievorschlag umfasst u. a. Festlegung auf das einzusetzende Produkt (beispielsweise Nabelschnurblut-Präparat als Fertigarzneimittel), Zeitpunkt, Ablauf und Dauer der Behandlung sowie Anzahl, Dosierung und Gabe des oder der Produkte und mögliche Maßnahmen für den Fall eines Rückfalls.

Weiterhin beschreibt im Sinne der Erfindung das Kriterium Dringlichkeit nach definierter Klassifizierung, die für alle Nutzer der Datenbank oder Plattform verbindlich festgelegte Priorisierung der Suche und Zuordnung eines geeigneten Präparates für einen bestimmten Patienten gegenüber der parallel laufenden Suche für einen anderen Patienten, die möglicherweise aufgrund ihrer genetischen Typisierungen für das gleiche Produkt im Bestand in Frage kommen. Die Klassifizierungstabelle kann von einem Koordinator festgelegt werden und orientiert sich an der medizinischen Dringlichkeit, mit der der Patient das Präparat benötigt

Vorteilhafterweise können die Parameter, die beispielsweise für einen bestimmten Patienten wichtig sind und die folglich für die Suche nach einem geeigneten Präparat wichtig sind vor der Suche von dem behandelndem Arzt oder der Klinik vordefiniert werden, wodurch eine effiziente und automatisierte Suchabwicklung möglich ist.

Es wird beispielsweise die Information über die behandelnde Klinik nicht nur für die Qualitätssicherung des Prozesses protokolliert, sondern als notwendige Information im Vorfeld erhoben, ohne die der Suchprozess nicht beginnen kann. Weiterhin erhebt die bevorzugte Ausführungsform automatisch eine Statistik pro Klinik über Anzahl und Art der Transplantationen, wodurch die Beurteilung einer Klinik bezüglich ihrer Eignung für eine Transplantation vereinfacht wird. Hierdurch können besonders einfach Kliniken ausgeschlossen werden, die wenig bis keine Erfahrung mit Transplantationen haben.

Die Dringlichkeit des Falles, beispielsweise der Transplantation, wird bei der Priorisierung von Präparaten im Konfliktfall berücksichtigt. Es ist für die Automatisierung der Vorgänge vorteilhaft, wenn potentiell auftretende Konflikte bezüglich der Reservierung und Bestellung von NSBP gelöst werden können. Hierzu kann u. a. die Priorisierungsinformation verwendet werden. Weiterhin werden vorteilhafterweise auch Informationen, die für eine automatisierte Abrechnung notwendig sind, erfasst. Besonders bei der automatisierten Massenabwicklung ist dies eine zwingende Voraussetzung und vereinfacht die Automatisierung maßgeblich und ist somit eine wesentliche Reduzierung der Arbeitsschritte.

Während der bevorzugten automatischen Auswahl eines geeigneten Präparates, können vorteilhafterweise auf jeder Stufe Informationen bzw. Listen der geeigneten Präparate abgerufen werden. Hierdurch wird für den Koordinator oder den Fachmann, der die Suche durchführt, die Auswahl übersichtlich gestaltet.

Es ist vorgesehen dass die Ordnung der potentiellen Nabelschnur-Präparate wie folgt festgelegt ist:

$ML_{Präp}$ = Matchlevel entsprechend der HLA Übereinstimmung zwischen Präparat und Patient

$$ML_{Präp} := \begin{cases} 6 : HLA_{Präp} \text{ und } HLA_{Pat} \text{ matchen in } 6 \text{ von } 6 \text{ Werten und Blutgruppenverträglichkeit} \\ 5 : HLA_{Präp} \text{ und } HLA_{Pat} \text{ matchen in } 5 \text{ von } 6 \text{ Werten und Blutgruppenverträglichkeit} \\ 4 : HLA_{Präp} \text{ und } HLA_{Pat} \text{ matchen in } 4 \text{ von } 6 \text{ Werten und Blutgruppenverträglichkeit} \\ Pr\ddot{a}parat \text{ wird nicht berücksichtigt} : sonst \end{cases}$$

$ZF_{Präp}$ = der Zellfaktor definiert die benötigten Zellzahl pro kg Patientengewicht bei entsprechendem Matchlevel

$$ZF_{Präp} := \begin{cases} 3 \times 10^7 : ML_{Präp} = 6 \\ 4 \times 10^7 : ML_{Präp} = 5 \\ 5 \times 10^7 : ML_{Präp} = 4 \end{cases}$$

$OZ_{Präp}$ = Ordnungszahl eines Präparates mit der die Präparate entsprechend TNC und Matchlevel geordnet werden können

$$OZ_{Präp} := \frac{TNC_{Präp}}{ZF_{Präp}}$$

$SL_{Single}$ = Shortlist der zu berücksichtigenden Präparate für Einzeltransplantate

$$SL_{Single} := \left\{ p \in Pr\ddot{a}p \left| \frac{OZ_p}{KG_{Pat}} \geq \frac{1}{kg} \wedge ML_{Präp} \geq 4 \right. \right\}$$

Die Standardordnungen der Präparate in einer Shortlist erfolgen nach folgenden Kriterien:

Ordnung 1 = Reihung zuerst nach Matchlevel, dann nach Ordnungszahl, dann nach CD34⁺

$$\text{Ordnung 1 (SL)} := \left\{ p1 \in SL, p2 \in SL \left| \begin{array}{l} \textit{entweder } ML_{p1} > ML_{p2} \\ \textit{oder } ML_{p1} = ML_{p2} \wedge OZ_{p1} > OZ_{p2} \\ \textit{oder } ML_{p1} = ML_{p2} \wedge OZ_{p1} = OZ_{p2} \wedge CD34_{p1} \geq CD34_{p2} \end{array} \right. \right.$$

Ordnung 2 = Reihung zuerst nach Ordnungszahl, dann nach Matchlevel, dann nach CD34+

$$\text{Ordnung 2 (SL)} := \left\{ p1 \in SL, p2 \in SL \left| \begin{array}{l} \textit{entweder } OZ_{p1} > OZ_{p2} \\ \textit{oder } OZ_{p1} = OZ_{p2} \wedge ML_{p1} > ML_{p2} \\ \textit{oder } OZ_{p1} = OZ_{p2} \wedge ML_{p1} = ML_{p2} \wedge CD34^+_{p1} \geq CD34^+_{p2} \end{array} \right. \right.$$

Hierbei entspricht:

Präp = Nabelschnurblutpräparat

Pat = Patient

$HLA_{Pat}$ = HLA Werte des Patienten

$HLA_{Präp}$ = HLA Werte eines Präparates

$TNC_{Präp}$ = Anzahl der kernbehafteten Zellen eines Präparates

$KG_{Pat}$ = Körpergewicht des Patienten in kg

$CD34+_{Präp}$ = Anzahl der CD34+ Zellen eines Präparates

Die beanspruchte Ausführungsform ist demgemäß auch eine Kombination, bei der die genannten Elemente zur Erreichung eines technischen Gesamterfolges zusammenwirken, wodurch ein synergistischer Effekt entsteht, welcher sich in einer überraschend effizienten und schnellen Suche nach adäquaten Präparaten zeigt. Demgemäß können die Ordnungskriterien im Sinne der Erfindung auch als Suchkriterien bezeichnet werden. Vorteilhafterweise kann die Suche automatisch erfolgen, wodurch die Suche erheblich schneller ablaufen kann und keine Fehler durch an der Suche beteiligten Personen erfolgen. Vorteilhafterweise wird durch die Standardisierung des Suchverfahrens und die Kombination der Ordnungskriterien, eine automatisierte Massenabwicklung ermöglicht. Es kann jedoch auch bevorzugt sein, dass die Präparate zusätzlich nach sonstigen Ordnungskriterien priorisiert werden, beispielsweise nach den Mutationen in einem bestimmten Gen oder nach der Blutgruppe. Es kann auch bevorzugt sein, dass die Ordnung der Präparate anhand von Genom-, Proteom-, Transkriptom-, Epigenom- und/oder Metabolomdaten festgelegt wird. So können beispielsweise Präparate durch das System direkt aussortiert oder separat gelistet werden, die eine erhöhte Konzentration eines Biomarkers aufweisen und Rückschlüsse auf eine erhöhte oder erniedrigte Stoffwechselaktivität ermöglichen, die inkompatibel mit den charakteristischen Eigenschaften des Empfängers sind. Auch genetische Defekte, die ein erhöhtes Abstoßungsrisiko bedeuten, können durch das System so vor der Transplantation erkannt werden. Es kann vorteilhaft sein, dass die Genom-, Proteom-, Transkriptom-, Epigenom- und/oder Metabolomanalysen von Dienstleistern durchgeführt werden. Zum Einen verfügen spezialisierte Dienstleister über hochauflösende Analyseapparate und zum Anderen können so Kosten eingespart werden. Das Pooling der generierten Daten wird durch das System durchgeführt, das heißt, die generierten Daten können in das System eingegeben und von diesem auf einem Speichermedium gespeichert werden. Es können so alle Beteiligten, die mit dem System vernetzt sind, auf die Daten zugreifen. Die hochauflösenden Analysemethoden generieren sehr umfangreiche Daten, die Auskunft über eine Vielzahl von Faktoren geben und so eine Transplantation, beziehungsweise den Effekt der Transplantation auf den Empfänger detailliert analysieren können.

Die Suche nach einem geeigneten Präparat gliedert sich in mehrere Stufen. Die erste Stufe ist der "Basic Search". Nach einem Ausschlussprinzip erscheinen auf der so genannten "Long List", das heißt eine lange Liste, die vorteilhafterweise alle Präparate in Rangfolge, die in mindestens vier von sechs HLA Typisierungen übereinstimmen umfasst und sich gemäß der Blutgruppenzugehörigkeit nicht ausschließen, d.h. es ergibt sich ein Matchlevel entsprechend der HLA Übereinstimmungen zwischen Präparat und Patient. Die Klinik kann zu diesem Zeitpunkt auch Präparate aus NSB-Banken, die nicht registriert sind, in den "Basic Search" einspielen. Es kann auch bevorzugt sein, dass die Rangfolge der langen Liste anhand von Genom-, Proteom-, Transkriptom-, Epigenom- und/oder Metabolomdaten erstellt wird.

Die nächste Stufe ist der "Advanced Search", wobei eine zweigeteilte "Short List", das heißt eine kurze Liste, genutzt werden kann. Die Liste umfasst vorteilhafterweise mögliche Einzel-Transplantate (SingleCord View). Dies sind Präparate, die in der Korrelation der Ordnungskriterien HLA-Match, Patientengewicht und Anzahl so genannter kernhaltigen Zellen (TNC) sowie Anzahl an hämatopoetischen Zellen (CD34+) als einzelnes Transplantat in Frage kommen.

Es kann jedoch auch vorteilhaft sein, dass die Präparate anhand von Genom-, Proteom-, Transkriptom-, Epigenom- und/oder Metabolomdaten sortiert und geordnet werden. Die Korrelation basiert auf folgenden Kennzahlen. Bei einem HLA-Match von sechs aus sechs benötigt der Patient beispielsweise mindestens $3,0 \times 10^7$ TNC pro kg Körpergewicht des Patienten; d.h. hat der Patient ein Körpergewicht von beispielsweise 55 kg könnte das Präparat insgesamt über mindestens $1,65 \times 10^9$ kernhaltiger Zellen verfügen. Bei einem HLA-Match von fünf aus sechs benötigt der gleiche Patient beispielsweise mindestens $4,0 \times 10^7$ TNC pro kg Körpergewicht, wonach das Präparat beispielsweise bei einem Patientengewicht von 55 kg über mindestens $2,2 \times 10^9$ TNC verfügen könnte. Weiterhin könnte bei einem Match von 4 aus 6 HLA-Typen das Präparat über beispielsweise mindestens $5,0 \times 10^7$ TNC/kg also insgesamt über $2,75 \times 10^9$ TNC verfügen. Somit können vorteilhafterweise Ranglisten der identifizierten Präparate nach beispielsweise zwei wählbaren Kriterien erstellt werden: 1) höchster HLA-Match und nachfolgend höchste relative Zellzahl oder 2) höchste relative Zellzahl und nachfolgend höchster HLA-Match. Falls die ermittelten Präparate die gleiche Positionierung aufweisen, wird die weitergehende Rangfolge der Präparate durch die Höhe der CD34$^+$ Zellzahl festgelegt.

Durch die bevorzugte Ausführungsform und insbesondere durch die Kombination der Kriterien, die synergistisch zusammenwirken, kann das am besten passende Nabelschnurblutpräparat aus einem gegebenen Vorrat identifiziert werden und zum Versand vorbereitet werden. Vorteilhafterweise erfolgt das Auswahlverfahren des Präparates automatisiert. So ist es möglich, das zeitaufwändige manuelle Auswahlverfahren zu standardisieren und zu beschleunigen, welches heute eine zentrale Schwachstelle in der Lieferkette von Nabelschnurblutpräparaten darstellt.

Bevorzugt ist auch, dass folgende Ordnungskriterien und/oder Ausschlußkriterien herangezogen und individuell gewichtet werden:

- Präparate mit einer CD34$^+$ Zellzahl höher 10% der TNC Zellzahl

- Ausschluss von Präparaten, bei denen im CA (Colony Assay) weniger als 75% der CD34$^+$ Zellen überlebten bzw. aktiviert wurden

- Blutgruppenidentität

- Ethnische Identität

- Geschlecht

- Alter des Präparates

- Akkreditierungsstandard

- Ranking der NSB-Bank.

Durch die bevorzugte Ausführungsform kann sichergestellt werden, dass eine optimale Qualität der Präparate garantiert wird und so eine erfolgreiche Transplantation ermöglicht werden kann. Hierfür werden vorteilhafterweise Präparate mit einer CD34$^+$ Zellzahl höher 10% der TNC Zellzahl unterschiedlich gewichtet. Präparate, bei denen im CA (Colony Assay) weniger als 75% der CD34$^+$ Zellen überlebten bzw. aktiviert wurden werden ausgeschlossen, um eine hohe Anzahl an hämatopoetischen Stammzellen zu garantieren. Weitere Kriterien, wie Blutgruppenidentität, ethnische Identität und Geschlecht können weiterhin die Auswahl des Präparates einschränken. Des Weiteren können durch die Bestimmung des Alters des Präparates, alte Präparate ausgeschlossen werden, wodurch vorteilhafterweise nur Präparate zur Transplantation verwendet werden, die ein definiertes Alter nicht überschritten haben, wodurch eine überraschend hohe Qualität sichergestellt wird. Der Akkreditierungsstandard Ranking der NSB-Bank kann ebenfalls zur Auswahl herangezogen werden. Somit können Banken ausgeschlossen werden, die beispielsweise wenig Erfahrung mit der Lagerung oder Transplantation von Nabelschnurblut haben. Durch die Kombination der Ordnungs- und/oder Ausschlußkriterien ist eine qualitative Charakterisierung der Präparate möglich, wodurch eine Abstoßung der Präparate bei einer Transplantation reduziert ist und sichergestellt wird, dass ein Patient "das beste", das heißt das für ihn verträglichste Präparat erhält.

Vorteilhafterweise können Auswahlkriterien festgelegt werden, die die Suche nach einem geeigneten Präparat einfacher gestalten und außerdem die Auswahl eines Präparates vereinfachen können. Hierzu können auch Informationen über die Zuverlässigkeit und der Liefergeschwindigkeit der NSB genutzt werden, die vom System automatisiert erhoben werden.

Diese zusätzlichen Ordnungskriterien können im Rahmen der Klinikpolitik einmalig vergeben oder in jedem Einzelfall neu priorisiert werden. Die Priorisierung entscheidet über die Feinauswahl im finalen Ranking der Präparate für die möglichen Lösungen.

Es ist ebenfalls bevorzugt, dass die bevorzugte Ausführungsform zur Vermittlung von Doppel- oder Mehrfachtransplantationen (Multicord) eingesetzt wird. Hierdurch ist es möglich abhängig von der benötigten Zellzahl, eine Doppel- oder Mehrfachtransplantation durchzuführen. Das heißt, benötigt der Patient mehr Zellen, als durch ein passendes Präparat zur Verfügung gestellt werden können, kann ein weiteres passendes Präparat automatisch gesucht werden.

Bevorzugt ist weiterhin, dass die Auswahl der Multicord-Präparate nach folgenden Ordnungskriterien erfolgt, $ML_{P1P2}$ = Verträglichkeit von 2 Präparaten untereinander:

$$ML_{P1P2} := \begin{cases} 6 : HLA_{Pr\ddot{a}p1}\ und\ HLA_{Pr\ddot{a}p2}\ matchen\ in\ 6\ von\ 6\ Werten\ und\ Blutgruppenvertr\ddot{a}glichkeit \\ 5 : HLA_{Pr\ddot{a}p1}\ und\ HLA_{Pr\ddot{a}p2}\ matchen\ in\ 5\ von\ 6\ Werten\ und\ Blutgruppenvertr\ddot{a}glichkeit \\ 4 : HLA_{Pr\ddot{a}p1}\ und\ HLA_{Pr\ddot{a}p2}\ matchen\ in\ 4\ von\ 6\ Werten\ und\ Blutgruppenvertr\ddot{a}glichkeit \\ Pr\ddot{a}parat\ wird\ nicht\ ber\ddot{u}cksichtigt : sonst \end{cases}$$

$GL_{Multi}$ = Grundliste zur Ermittlung der Auswahlliste für Multipräparate

$$GL_{Multi} := \left\{ p \in Pr\ddot{a}p \left| \frac{OZ_p}{KG_{Pat}} < \frac{1}{kg} \wedge ML_{Pr\ddot{a}p} \geq 4 \right. \right\}$$

$SL_{Multi}$ = Shortlist der zu berücksichtigenden Präparate für Multitransplantate

$$SL_{Multi} := \left\{ p1 \in GL_{Multi}, p2 \in GL_{Multi} \left| ML_{p1p2} \geq 4 \wedge \frac{OZ_{p1} + OZ_{p2}}{KG_{Pat}} \geq 1 \right. \right\}$$

Die bevorzugte Ausführungsform bietet einen zweiten Teil der "Short List" den MultiCord View mit Präparaten an, die zueinander gematched, das heißt zueinander passend, sind. Vorteilhafterweise ist bereits die Eignung der verschiedenen Präparate zueinander definiert, wodurch vorteilhafterweise keine Unverträglichkeit zwischen mehreren dem Patienten verabreichten Präparaten entsteht.

Vorteilhafterweise sind auf der Short-List Präparate aufgeführt, die bislang noch gar nicht alleine oder in Kombination in Betracht kamen und wodurch die benötigte Zellzahl entweder erreicht oder sogar überschritten wird. Bei MultiCord Präparaten ist dasjenige (Teil-)Präparat als vorteilhaft (als das "erste") anzusehen, dass die höhere CD34+ Zellzahl aufweist. Auf der Short List erscheint zu jedem SingleCord und MultiCord ein vorläufiges Budget , das die Kosten hinsichtlich eines Präparates nach Standardwerten entsprechend dem Status der Präparates kalkuliert. Es kann auch vorteilhaft sein, die MultiCord Präparate anhand von Genom-, Proteom-, Transkriptom-, Epigenom- und/oder Metabolomdaten auszusuchen. Die Präparate werden bevorzugt vor einer Transplantation charakterisiert, indem Genom-, Proteom-, Transkriptom-, Epigenom- und/oder Metabolomanalysen durchgeführt werden und die Daten in das System eingefügt und gespeichert werden. Hierdurch ist eine detaillierte Charakterisierung der Präparate, insbesondere auf molekularer Ebene möglich. Es hat sich überraschenderweise gezeigt, dass das System mithilfe dieser Charakterisierung die Zahl der Abstoßungsreaktion maßgeblich reduzieren kann.

Die Suchresultate können vorteilhafterweise in einem "Compare View" mit bis zu vier Präparaten, die in übersichtlicher Weise mit den Patientendaten verglichen werden können, dargestellt werden. Der "Compare View" vergleicht alle Daten des Unit Reports mit den Daten des Patienten.

Die letzte Stufe des Auswahlverfahrens sind die Lösungsvorschläge, die vorteilhafterweise einem behandelnden Arzt übersichtlich präsentiert werden. Hierbei umfassen die Vorschläge vorteilhafterweise Einzelpräparate und/oder Multi Cord Präparate. Es ist die abschließende Entscheidung des Arztes, ob und für welches SingleCord oder MultiCord er sich entscheidet.

Für jeden Lösungsvorschlag können vorteilhafterweise Ordner/Akte mit vier Blättern angelegt werden. Diese Akte ist Arbeits- und Kommunikationsinstrument für die Zusammenarbeit des Koordinators mit dem behandelnden Arzt bzw. hinsichtlich des Patienten und der Klinikverwaltung.

Beispielsweise kann dieser Ordner wie folgt aufgebaut sein:

Blatt 1 kann ein Arbeitsblatt oder Laufzettel auf dem das oder die Präparate neben den Patientendaten gezeigt werden und auf der die notwendigen weiteren Schritte, die bis zur Transplantation bearbeitet werden können darstellen. Dies sind vor allem die Anforderung von HLA-Typing, DNA-Samples, CAs, Genom-, Proteom-, Transkriptom-, Epigenom- und/oder Metabolomanalysen aber auch direkte Kontaktaufnahme mit der betreffenden NSB-Bank, Reservierungen bis hin zur verbindlichen Bestellung, Transportlogistik und Fakturierung bis hin zur Übergabe an die Klinik Administration.

Blatt 2 des Lösungsvorschlags kann den oder die vollständigen Unit Reports enthalten.

Blatt 3 des Lösungsvorschlags kann zur Dokumentation der Entscheidung dienen; er fasst die Entscheidungskriterien des Arztes zusammen, stellt das abschließende Budget fest und wird von Arzt abgezeichnet. Auf diesem Blatt kann der Arzt auf andere Lösungsvorschläge verweisen, die alternativ zur Anwendung kommen, wenn sich der gewünschte Lösungsvorschlag aufgrund von Ereignissen nicht oder nicht mehr umsetzen lässt.

Blatt 4 kann eine übersichtliche Darstellung hinsichtlich des Präparates, des Zeitablaufes und der Transplantation im Allgemeinen, das dem Arzt für das Patientengespräch bzw. dem Patienten zur Information zu Verfügung steht umfassen.

Weiterhin können vorteilhafterweise vorgeschlagenen Lösungen im Anhang der Akte dokumentiert werden, sollte sich die erste Lösung nicht Verwirklichen lassen (z.B. Transportschäden oder Verlust eines Präparates aus der ersten Lösung).

Die Blätter aus dem Lösungsvorschlag stehen der Klinik für den weiteren Verlauf wie Abrechnung und Nachverfolgung der Transplantation wie z.B. der Anamnese des Patienten zur Verfügung und werden zu gegebenen Zeitpunkt an die betreffende NSB-Bank bzw. NSB-Banken weitergeleitet.

Während des gesamten Verlaufs bis zur Nachsorge des Patienten, werden vorteilhafterweise Daten von neu registrierten NSB-Präparaten der Klinik automatisch übermittelt und bewertet hinsichtlich des Rankings auf der Long und Short List sowie bei den vorgeschlagenen Lösungen.

Damit ist eine dynamische Verbesserung der Lösungsvorschläge bzw. eine Nachbehandlung des Patienten auf dem jeweils jüngsten Datenbestand gesichert.

In einer bevorzugten Ausführungsform wird ein für die allogene Transplantation geeignetes Zellpräparat ausgewählt. Bei einer Allotransplantation stammt das transplantierte Gewebe nicht vom Empfänger selbst, sondern von einem Spender derselben biologischen Art. Um eine schwerwiegende oder sogar tödliche Abstoßung des Fremdgewebes zu vermeiden, ist für die erfolgreiche allogene Transplantation die möglichst vollständige Übereinstimmung der vom Immunsystem erkannten Merkmale mit dem Empfängergewebe erforderlich. Es hat sich hierfür als vorteilhaft herausgestellt, dass eine Genom-, Proteom-, Transkriptom-, Epigenom- und/oder Metabolomanalyse durchgeführt wird. Durch diese hochauflösenden Analysen wird das Präparat sowie der Empfänger detailliert durch das System charakterisiert. Es ist weiterhin bevorzugt, dass zur Überwachung einer Abstoßung, vor und nach der Transplantation Proben für die genannten Analysen entnommen werden, da hierdurch eine konstante Beobachtung durch das System und eine schnelle Diagnose der Abstoßung möglich ist. Durch die bevorzugte Ausführungsform kann anhand von den vorgebenden Parameter die Suche nach einem geeigneten, das heißt passenden Präparat einfach, schnell und vorteilhafterweise automatisiert durchgeführt werden, wodurch überraschenderweise die die Gefahr einer Abstoßungsreaktion minimiert ist und einer erfolgreichen Transplantation nichts im Wege steht.

In einer weiteren bevorzugten Ausführungsform erfolgt die automatische und vollständige Auswahl für Singlecord- oder Multicord-Transplantate. Hierbei werden dem behandelnden Arzt und/oder dem Koordinator entsprechende Präparate vorgeschlagen, die basierend auf den Parametern zueinander passen und keine Abstoßungsreaktionen generieren. Es werden vorteilhafterweise die zu einander und zu dem Patienten passenden Präparate entsprechend dargestellt, um so die Auswahl erheblich zu vereinfachen und zu beschleunigen. Der behandelnde Arzt kann dementsprechend beide Auswahlmöglichkeiten dargestellt bekommen und kann selbst beurteilen, ob eine Multicord oder Singlecord Transplantation erfolgen soll. Überraschenderweise können durch eine automatische Auswahl Fehler vermieden werden und Singlecord- und/oder Multicord-Transplantate dem behandelnden Arzt präsentiert werden. Vorteilhafterweise erfolgt die Präsentation in einer übersichtlichen Weise, wodurch die Auswahl der Präparate durch den Arzt vereinfacht wird:

Es ist bevorzugt, dass die Suchkriterien an die eingetragenen Kriterien und/oder Parameter angepasst werden. Bei der Auswahl des geeigneten Präparates wird der aktuelle Typisierungsstatus des NSBP berücksichtigt. Das heißt es wird beispielsweise beurteilt, welche zusätzlichen Untersuchungen, umfassend Genom-, Proteom-, Transkriptom-, Epigenom- und/oder Metabolomanalysen usw. notwendig sind, damit das Präparat als geeignet bestätigt werden und transplantiert werden kann. Hierzu nutzt die bevorzugte Ausführungsform automatisiert erhobene Statistiken über die erwarteten Kosten und die benötigte Zeit. Dies ist insbesondere bei zeitkritischen Einsatzszenarien unbedingt notwendig und beschleunigt den Auswahlprozess maßgeblich. Grundsätzlich ist die Erweiterbarkeit des Datenschemas von NSBP und Patient vorteilhaft, um weitere Suchkriterien an den zukünftigen Stand der Technik angleichen zu können.

Weiterhin ist bevorzugt, dass für die Darstellung der über die Suchkriterien erlangten Ergebnisse eine Matrix verwendet wird und die Ergebnisse visuell dargestellt werden. Hierzu bietet die bevorzugte Ausführungsform eine visuelle Orientierung über die besten Suchergebnisse gemäß der aktuell gewählten Suchparameter an. Die Suchergebnisse werden hierzu in einer Matrix angeordnet und visualisiert. Die Matrix kann entsprechend der verschiedenen Kriterien dynamisch umsortiert werden. Die Passgenauigkeit anhand der voreingestellten Suchkriterien wird farbig angezeigt. Im Sinne der Erfindung kann die Matrix als Heat Map bezeichnet werden, bei welcher Daten eines Parameters als Farben in einer zwei-dimensionalen Darstellung aufgeführt werden.

Weiterhin ist bevorzugt, dass für die Beurteilung des Status der Ordnungskriterienermittlung Statistiken über die zu erwartenden Kosten und die zu benötigende Zeit verwendet werden. Vorteilhafterweise können Statistiken für die Suche nach einem geeigneten Präparat für die Ordnungskriterienermittlung herangezogen werden. Hierfür können beispielsweise die zu erwartenden Kosten, die benötigte Zeit, die erfolgreichen Transplantationen einer Klinik und die noch durchzuführenden Untersuchungen in die Auswahl eines Präparates, beziehungsweise in die Ordnung, die ein Präparat einnimmt mit einfließen. Hierdurch können Präparate schneller beurteilt und entsprechend geordnet werden. Außerdem ist eine kosten- und zeitreduzierende Suche möglich.

Somit können automatische und vollständige Lösungsvorschläge für SingleCord- oder MultiCord-Transplantate erstellt werden. Der Koordinator und der Arzt können sich vorteilhafterweise auf die Eignung der verschiedenen gut definierten und dokumentierten Lösungsvorschläge konzentrieren. Hierbei ist bevorzugt, dass die Koordination zwischen Klinik, Transplantationszentrum und behandelndem Arzt durch die bevorzugte Ausführungsform erfolgt. So kann sichergestellt werden, dass eine verlässliche Kommunikation zwischen der Klinik, das heißt ggf. dem behandelnden Arzt und dem Transplantationszentrum erfolgt. Die Suchparameter sowie die Ergebnisse werden übersichtlich präsentiert, wodurch die Auswahl erheblich vereinfacht ist. Ebenfalls sind die Parameter, nach derer Basis die Suche erfolgt variabel und können an den Patienten und/oder das gesuchte Präparat angepasst werden. Dies ist ein großer Fortschritt gegenüber der bisherigen Situation, bei der die Koordinatoren gezwungen sind, mögliche Transplantate zu einem sehr frühen Zeitpunkt und nach unterschiedlichen Kriterien zu bewerten. Dies führt heute zu unbefriedigenden Ergebnissen und ist sehr zeit- und personalaufwendig. Somit kann durch die bevorzugte Ausführungsform in einer kurzen Zeit ein oder mehrere passende Präparate gesucht und bestellt werden.

Die Erfindung soll im Folgenden beispielhaft erläutert werden, ohne jedoch auf die Beispiele begrenzt zu sein.

Das so genannte Matching eines Nabelschnurpräparates für den Patienten Kurt Werner erfolgt zwischen der behandelnden Transplantationsklinik und einer Nabelschnurblutbank durch das beschriebene System. Sobald ein gemäß des HLA Matches und anderer Daten passendes Nabelschnurblutpräparat für Kurt Werner bestellt wird, löst das System automatisch die Bestellung eines Aliquots des Nabelschnurblutpräparates bei der Nabelschnurblutbank und eine Blut- oder Gewebeprobe des Patienten vor der Konditionierung bzw. Transplantation aus. Die beiden Proben werden an das Labor verschickt, das ein Biomarkermuster des Aliquots bzw. der Patienten Blutprobe erstellt. Das heißt, es werden metabolische Analysen durchgeführt, die die Metabolite bestimmen und so Rückschlüsse auf Stoffwechselaktivitäten zulassen. Hierdurch können beispielsweise Stoffwechselerkrankungen einfach und schnell erkannt werden.

Diese Ausgangsprofile werden automatisch im System in einer hoch verschlüsselten Patientenakte neben der Anamnese und den üblichen Standard Diagnosewerten des Patienten, die mit der Bestellung des Präparates von dem Transplantationszentrum übermittelt werden, abgelegt. In der Nachfolge der Transplantation werden zu bestimmten Zeitpunkten mindesten im Verlauf der gesetzlich geforderten fünf Jahre, automatisch vom System die notwendigen Proben, beispielsweise Blutproben des Patienten Kurt Werner bestellt und nach Profilierung des Biomarker Musters und den Analyse der metabolischen Daten durch das zuständige Labor automatisch in der Patientenakte neben den üblichen Standard Diagnosewerten des Patienten und den Berichten des weiteren Verlaufs der Krankheit in der Datenbank des Systems abgelegt. Das System stellt automatisch alle Patientenwerte der zuständigen Klinik und dem Patienten im vollen Umfang zur Verfügung, der betreffenden Nabelschnurblutbank im definierten Umfang, um die gesetzlichen Vorgaben zu erfüllen und mittels entsprechender Einstellungen kann das System in anonymisierter Form aus der Gesamtheit der relevanten Patientendaten statistische und wissenschaftliche Analysen erstellen. Diese sind u. a. Grundlage für die Definition und Validierung von Biomarkern oder Metaboliten.

Damit ist dieses System erstmalig in der Lage zwischen allen Partnern, Bank/Register, Klinik, Dienstleistern und Akademie die Daten zu sammeln, zu verteilen und aufzubereiten.

Die Erfindung wird nunmehr anhand von Figuren beispielhaft erläutert, ohne auf die Beispiele begrenzt zu sein. Es zeigen:

Fig. 1    Das Basis-Datenmodell

Fig. 2    Den Prozessablauf

Fig. 3    Die Systemarchitektur

Fig. 4    Eine Nachverfolgung über das System

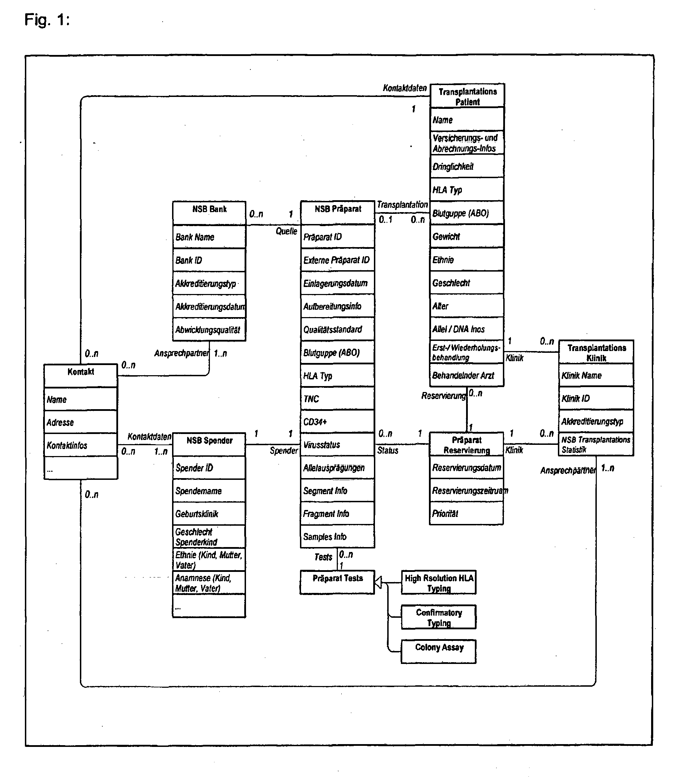

Fig. 1 zeigt eine beispielhafte Darstellung einer bevorzugten Ausführungsform des Basis-Datenmodell. Bei einem ersten Kontakt, können die Daten einer Person aufgenommen werden, wie beispielsweise Name, Adresse und sonstige Kontaktinfos. Es kann beispielsweise ein NSB Spender sein, wobei hier noch weitere Daten in die Datenbank eingegeben werden können (umfassend Geburtsklinik und Anamnese von der Mutter, Vater und/oder dem Kind). Vorteilhafterweise können auch Daten der NSB-Bank erfasst und gespeichert werden. Vorteilhafte Daten umfassen Abwicklungsqualität und eine spezifische Bank ID. Die Daten der NSB Präparate umfassen vorteilhafterweise HLA Typ, TNC Nummer, oder Virusstatus. Anhand dieser Information kann ein Präparat exakt charakterisiert werden, wobei zusätzliche Information über das Präparat durch weitere Tests (umfassend "High Resolution HLA Typing", hochauflösende HLA Typisierung oder Colony Assays) bestimmt werden. Es hat sich überraschenderweise gezeigt, dass insbesondere Genom-, Proteom-, Transkriptom-, Epigenom- und/oder Metabolomanalysen eine umfangreiche und detaillierte Charakterisierung der Präparate bewirken. Es können so Informationen umfassend Methylierungsstatus definierter Gene, Expression von Biomarkern oder Gen-/Chromosomenaberrationen bestimmt werden. Das Präparat wird durch die Tests untersucht und die Qualität kann einfach beurteilt werden. Vorteilhafterweise vergleicht das System die Daten des Präparates mit den Daten eines Transplantations Patienten, das heißt es kann beispielsweise die Blutgruppe und die Dringlichkeit verglichen werden. Vorteilhafterweise können ebenfalls die Genom-, Proteom-, Transkriptom-, Epigenom- und/oder Metabolomdaten des Empfängers, d. h. der Patienten durch das System analysiert werden. Basierend auf dem Vergleich kann das Präparat vorteilhafterweise für den Patienten von der Transplantations Klinik reserviert werden. Dies kann beispielsweise von einem Koordinator oder einem behandelnden Arzt der Klinik erfolgen. Vorteilhafterweise können Daten der Klinik aufgenommen und in eine bevorzugte Datenbank gespeichert werden. Hierbei umfassen die Daten beispielsweise Klinik ID oder Akkreditierungstyp. Weiterhin ermöglicht das System die Überwachung der Transplantation, indem zusätzlich nach der Transplantation in bevorzugt regelmäßigen Abständen Proben für Genom-, Proteom-, Transkriptom-, Epigenom-und/oder Metabolomanalysen entnommen werden. Hierdurch kann der Verlauf der Transplantation exakt beschrieben und Komplikationen frühzeitig erkannt werden.

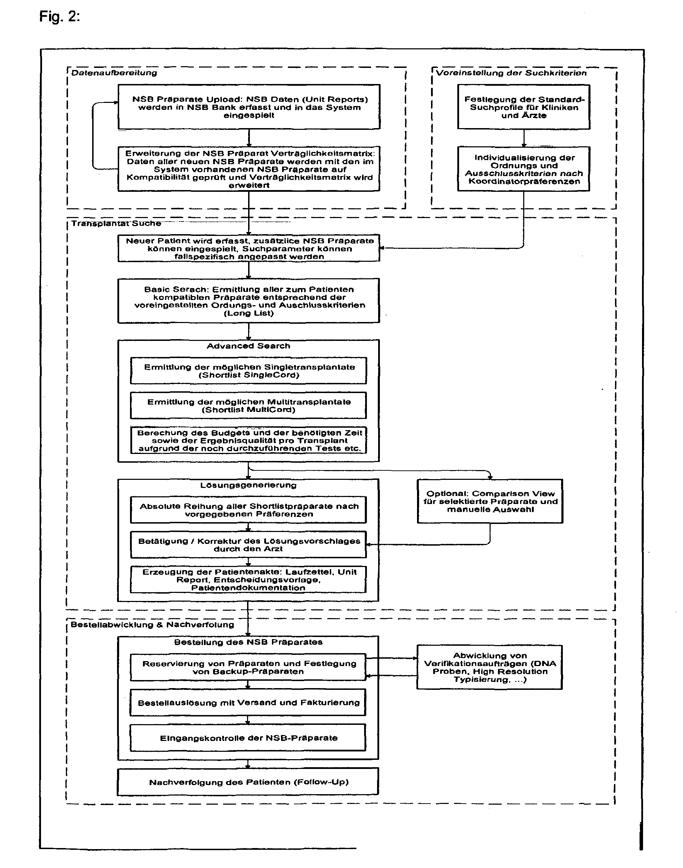

Fig. 2 zeigt eine beispielhafte Darstellung des Prozessablaufes. Bei der Datenaufbereitung können neue NSB Präparate in der NSB-Bank erfasst werden und in das bevorzugte System eingespielt werden. Vorteilhafterweise können die neu erfassten Präparate mit den im System vorhandenen NSB Präparaten auf Kompatibilität verglichen werden. Vorteilhafterweise kann dies beispielsweise über eine Verträglichkeitsmatrix erfolgen. Wenn ein NSB Präparat gesucht wird, können die Standard-Suchprofile für Kliniken und Ärzte verwendet werden, wobei vorteilhafterweise auch eine Individualisierung der Ordnungs- und Ausschlusskriterien nach Koordinationspräferenz möglich sein kann. Die Suche nach einem für einen Transplantations Patienten passenden NSB Präparat kann weiterhin fallspezifisch angepasst werden. Es kann eine sogenannte Basic Search erfolgen, bei der vorteilhafterweise alle der zum Patienten kompatiblen NSB-Präparate entsprechend der voreingestellten Ordnungs- und/oder Ausschlusskriterien gesucht werden (Long List). Des Weiteren kann es vorteilhaft sein, eine sogenannte Advanced Search durchzuführen, die die möglichen Singletransplantate (Singlecord) und/oder Multitransplantate (MultiCord) ermittelt. Außerdem könnte vorteilhafterweise das benötigte Budget, die benötigte Zeit, sowie die Ergebnisqualität pro Transplantat aufgrund der noch durchzuführenden Tests aufgeführt werden. Hierbei können die gefundenen Präparate in einer Vergleichsansicht (Comparison View) miteinander verglichen werden, wobei auch einzelne Präparate miteinander verglichen werden können. Vorteilhafterweise kann eine Lösungsgenerierung erfolgen, die die Shortlistpräparate nach den vorgegebenen Referenzen aneinander reiht. Dem zu behandelnde Arzt kann so eine übersichtliche Darstellung der Präparate präsentiert werden, wobei vorteilhafterweise der generierte Lösungsvorschlag von dem Arzt bestätigt oder korrigiert werden kann. Die so generierte Lösung kann in die Patientenakte des Transplantationspatienten aufgenommen, umfassend die Laufzettel, Unit Report, Entscheidungsvorlage und Patientendokumentation. So können vorteilhafterweise in einer Akte alle für eine NSB Transplantation relevante Informationen gespeichert werden. Sobald die Transplantationsklinik, beziehungsweise der behandelnde Arzt ein oder mehrere NSB-Präparate ausgewählte haben, kann das Präparat bei der NSB-Bank bestellt werden.

Vorteilhafterweise können die durch die Suche gefundenen Präparate für einen Patienten oder für eine Klinik reserviert werden, wobei vorteilhafterweise Backup-Präparate festgelegt werden können, falls die gewählten Präparate nicht zur Verfügung stehen. Weiterhin können zusätzliche Verifikationen (umfassend DNA Proben, High Resolution Typisierung oder Genom-, Proteom-, Transkriptom-, Epigenom- und/oder Metabolomanalysen) die Qualität und Kompatibilität der Präparate sicherstellten. Nach einer Auswahl, können die Präparate bestellt und geliefert werden. Vorteilhafterweise erfolgt über die Präparate eine Eingangskontrolle bei der Klinik. Nach erfolgreicher Transplantation kann vorteilhafterweise eine Nachverfolgung des Patienten (Follow-up) erfolgen.

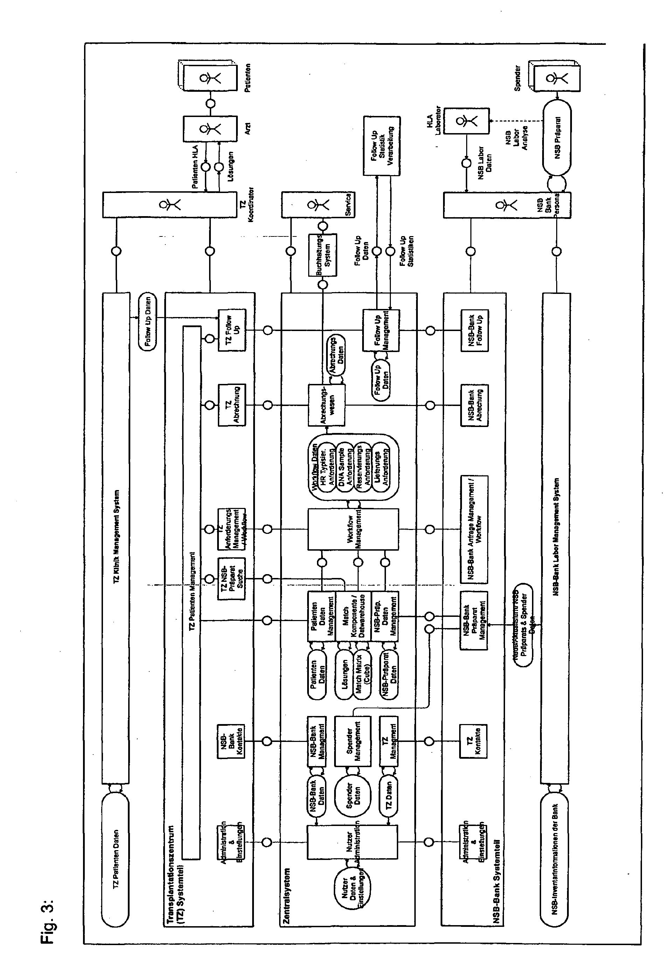

Fig. 3 zeigt eine beispielhafte Darstellung der bevorzugten Systemarchitektur. Die Darstellung zeigt einen schematischen Aufbau eines bevorzugten datenverarbeitenden Systemteils. Die bevorzugte Ausführungsform des Systems kann in 3 Teilbereiche Zentralsystem, TC (Transplantationszentrum) Systemteil und Nabelschnurblutbank (NSB-Bank) Teilsystem gegliedert sein. Das System ist vorteilhafterweise über das Inter- und Intranet nutzbar, wobei die einzelnen Teile über kabellose oder verkabelte Verbindungen miteinander kommunizieren und Daten austauschen können. Es wird gezeigt, dass NSB-Präparate von Spendern geliefert und durch ein HLA Labor analysiert werden können. Es kann auch bevorzugt sein, dass die Präparate von externen Dienstleister analysiert werden. Beispielsweise können Proben der NSB-Präparate an diese geschickt werden und eine Genom-Proteom-, Transkriptom-, Epigenom- und/oder Metabolomanalysen durchgeführt werden. Die dort generierten Daten werden beispielsweise an die NSB-Bank übermittel und in das System eingepflegt und gespeichert. Die Präparate können vorteilhafterweise von der NSB-Bank physikalisch aufbereitet und gelagert werden, wobei die gewonnenen Daten über das NSB-Präparat vorteilhafterweise bei der NSB-Bank in einem Labor Management System verwaltet werden können. Informationen ausgewählter NSB-Präparate können beispielsweise als Datensätze dezentral und inkrementell in das Zentralsystem überspielt werden. Der Nabelschnurblutbank bietet das bevorzugte System vorteilhafterweise die Möglichkeit zur Administration, Einsicht von Kontakten der Transplantationszentrum (TZ) Ansprechpartner, Verwaltung der eingespielten NSB-Präparate, komfortable Abarbeitung von Anfragen und Workflow Verfolgung, Verwaltung der kompletten Abrechnung von NSB-Präparat Lieferungen und Dienstleistungen sowie die Verwaltung von Follow-Up Informationen. Auch kann es vorteilhaft sein, wenn die NSB-Bank die Beauftragung zur Entnahme von Proben über das System, an beispielsweise die Klinik übermittelt. Weiterhin ist in Fig. 3 erkennbar, dass ein Arzt die HLA-Werte von Patienten ermitteln kann und beispielsweise an den TZ Koordinator zur Suche von NSB-Präparaten nebst weiterer Informationen übergeben kann. Der Koordinator kann beispielsweise eine systemgestützte Suche durchführen und liefert Lösungsvorschläge und vorteilhafterweise NSB-Präparate zur Transplantation. Die Patientendaten können vom Transplantationszentrum beziehungsweise der Klinik in einem eigenen Management System verwaltet werden. Vorteilhafterweise können Follow-Up Informationen nach der Transplantation hieraus an das Zentralsystem weitergegeben werden. Dem Transplantationszentrum kann das System beispielsweise die Möglichkeit zur Administration, Einsicht von Kontakten der NSB-Bank Ansprechpartner, Suche nach NSB-Präparaten, komfortable Beauftragung und Kontrolle von Anfragen und Workflow Verfolgung, Verwaltung der kompletten Abrechnung von NSB-Präparat Lieferungen und Dienstleistungen sowie die Verwaltung von Follow-Up Informationen bieten. Das Zentralsystem kann vorteilhafterweise einen gesicherten Zugang zu den Daten, die zur Wahrung der Datensicherheit verschlüsselt abgelegt sind (z.B. in einem Datenbanksystem) ermöglichen. Die Nutzerdaten und deren Einstellungen können vorteilhafterweie zentral abgelegt werden, so dass diese sitzungsübergreifend zur Verfügung stehen. Die Verwaltung der Zentralkomponente (umfassend Einrichten neuer Nutzer, Kliniken, NSB-Banken) kann beispielsweise von einem Servicepersonal vorgenommen werden. Von der NSB-Bank hochgespielte Informationen können im Spendermanagement und dem zentralen NSB-Präparatemanagement verwaltet werden. Die NSB-Daten können nach (beispielsweise modular austauschbaren) Ordnungskriterien vorgeordnet werden (beispielsweise durch ein Data Warehouse Cube), um vorteilhafterweise eine schnelle effiziente Suche auch bei komplexen Mehrfachtransplantationen zu garantieren. In der Matching Komponente können vorteilhafterweise modular unterschiedliche Matchingalgorithmen genutzt werden, um geeignete Lösungen automatisch oder halbautomatisch zu erzeugen. Alle Abläufe und Interaktionen zwischen den involvierten Parteien können durch die Workflowkomponente gesteuert werden. Des Weiteren können alle Transaktionen und Dienstleistungen durch die Abrechnungskomponente erfasst und ausgewertet werden. Vorteilhafterweise können aufbereitete Abrechungsinformationen an ein Buchhaltungssystem beispielsweise zur Rechnungslegung übergeben werden. Die Follow-Up Informationen können zentral verwaltet und an eine externe Zentralstelle übergeben werden, die beispielsweise Follow-Up Statistiken erzeugen und diese regelmäßig wieder übergeben kann.

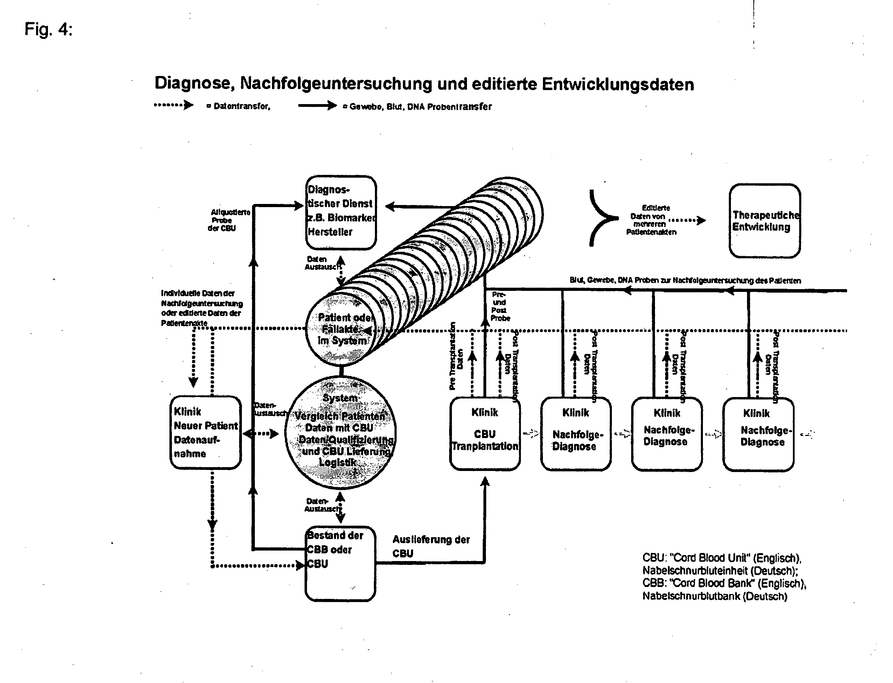

Fig. 4 zeigt die Nachverfolgung über das System. In einer Klinik wird ein neuer Patient aufgenommen und seine Daten werden in das System eingegeben und gespeichert. Die Daten liegen vorteilhafterweise in einer Patienten- oder Fallakte vor. Seine Daten werden von dem System mit dem Bestand an NSB-Präparaten verglichen. Sobald ein Match entdeckt wird, wird die Klinik benachrichtigt und der automatische Versand des Präparates ausgelöst. Der Match erfolgt bevorzugt nach definierten Parametern, umfassen HLA-Typen, Blutgruppe, Genom-, Transkriptom-, Metabolom- und/oder Epigenomdaten. Sobald ein beispielsweise gemäß des HLA Matches und anderer Daten passendes Nabelschnurblutpräparat für den Patienten bestellt wird, löst das System automatisch die Bestellung eines Aliquots des Nabelschnurblutpräparates bei der Nabelschnurblutbank und eine Blut- oder Gewebeprobe des Patienten vor der Konditionierung bzw. Transplantation aus. Die beiden Proben werden an ein Labor, beispielsweise ein diagnostischer Dienst (Biomarker Hersteller) verschickt, das ein Biomarkermuster des Aliquots bzw. der Patienten Blutprobe erstellt. Es kann auch vorteilhaft sein, zusätzlich zu den metabolischen Daten, eine Genom-, Epigenom- und/oder Transkriptomanalyse durchführen zu lassen. Diese Analysen stellen der Klinik sowie der NSB-Bank umfangreiche Informationen zur Verfügung, die eine detaillierte Charakterisierung des Patienten sowie des NSB-Präparates ermöglichen. Hierdurch können bereits vor einer Transplantation Inkompatibilitäten, die beispielsweise eine genetische Ursache haben identifiziert werden, wodurch Komplikationen vermieden werden können. Vorteilhafterweise werden diese Ausgangsprofile automatisch im System in einer hoch verschlüsselten Patientenakte neben der Anamnese und den üblichen Standard Diagnosewerten des Patienten, die mit der Bestellung des Präparates von dem Transplantationszentrum übermittelt werden, abgelegt. Nach der Transplantation beantragt das System in regelmäßigen oder unregelmäßigen Abständen die Entnahme und Analyse einer Probe (umfassend Blut, Gewebe, DNA, Urin, Speichel), um so auch nach der Transplantation die Werte des Patienten zu erfassen. Hierfür können beispielsweise Blutproben des Transplantationspatienten an diagnostische Dienste gesendet werden, die bevorzugt eine ähnliche Analyse wie vor der Transplantation durchführen (z. B. Genom-, Epigenom-, Metabolom- und/oder Transkriptomanalyse). Diese Daten werden automatisch in der Patientenakte des Systems gespeichert. Das System stellt automatisch alle Patientenwerte der zuständigen Klinik und dem Patienten im vollen Umfang zur Verfügung, der betreffenden Nabelschnurblutbank im definierten Umfang, um die gesetzlichen Vorgaben zu erfüllen und mittels entsprechender Einstellungen kann das System in anonymisierter Form aus der Gesamtheit der relevanten Patientendaten statistische und wissenschaftliche Analysen erstellen. Hierdurch kann die Transplantationseffizienz verbessert werden oder Risikogruppen schneller identifiziert werden. Es kann beispielsweise eine Korrelation der Genom-, Epigenom-, Metabolom und/oder Transkriptomdaten mit einer auftretenden Abstoßungsreaktion festgestellt werden, wodurch ggf. Faktoren identifiziert werden können, die eine Transplantation erschweren. Auch lässt sich der Einfluss von Medikamenten auf den Empfänger durch eine Erhebung der Genom-, Epigenom-, Metabolom und/oder Transkriptomdaten vor und nach der Transplantation besser beobachten, da die Ausgangsdaten mit den nach der Transplantation auftretenden Daten verglichen werden können Hieraus können neue Transplantationsstrategien abgeleitet und die Effizienz der Transplantation verbessert werden. Damit ist dieses System erstmalig in der Lage, durch eine Erhebung von Genom-, Epigenom-, Metabolom und/oder Transkriptomdaten vor und nach der Transplantation zwischen allen Partnern, Bank/Register, Klinik, Dienstleistern und Akademie die Daten zu sammeln, zu verteilen und aufzubereiten.

**Patentansprüche**

1. Verfahren zur Auswahl mindestens eines für die allogene Transplantation geeigneten Nabelschnurblut-Präparates, **dadurch gekennzeichnet, dass**
   das Verfahren die folgenden Schritte umfasst:

   - Eingabe von Erfahrungsdaten von Nabelschnurblut-Präparaten in einen Computer und Speicherung auf einem Speichermedium,
   - Eingabe von Anfragedaten eines potentiellen Empfängers des Nabelschnur-Präparats und Speicherung dieser auf einem Speichermedium,
   - Voreinstellung von Suchkriterien, insbesondere der Speicherung der Suchkriterien auf einem Speichermedium und/oder einer Verarbeitungseinheit,
   - Recherche, wobei die Recherche einen Vergleich der Erfahrungsdaten mit , den Anfragedaten umfasst und wobei ein Entnahmezentrum, eine Lagerstätte, eine Klinik, ein Transplantationszentrum und/oder eine Forschungseinrichtung über verkabelte und/oder kabellose Verbindungen auf einer oder mehreren Verarbeitungseinheiten, insbesondere Computer, medizinische Systeme, Speichervorrichtungen und/oder speziellen Prozessoren miteinander kommunizieren und über ein Netzwerk bestehend aus den mehreren Verarbeitungseinheiten, durch welche Daten ausgetauscht werden, miteinander verbunden sind und
   - automatische Auswertung des Rechercheergebnisses, wobei

   eine Rangliste ($SL_{single}$) der identifizierten Präparate für die Auswahl von mindestens einem für die allogene Transplantation geeigneten Nabelschnurblut-Präparates anhand folgender Ordnung erstellt wird:

$$ML_{Präp} = \begin{cases} 6: HLA_{Präp} \text{ und } HLA_{Pat} \text{ matchen in } 6 \text{ von } 6 \text{ Werten und Blutgruppenverträglichkeit} \\ 5: HLA_{Präp} \text{ und } HLA_{Pat} \text{ matchen in } 5 \text{ von } 6 \text{ Werten und Blutgruppenverträglichkeit} \\ 4: HLA_{Präp} \text{ und } HLA_{Pat} \text{ matchen in } 4 \text{ von } 6 \text{ Werten und Blutgruppenverträglichkeit} \\ \text{Pr} \ddot{a}parat \text{ wird nicht berücksichtigt}: sonst \end{cases}$$

$ZF_{Präp}$ = der Zellfaktor definiert die benötigten Zellzahl pro kg Patientengewicht bei entsprechendem Matchlevel

$$ZF_{Präp} := \begin{cases} 3\times10^{7}: ML_{Präp} = 6 \\ 4\times10^{7}: ML_{Präp} = 5 \\ 5\times10^{7}: ML_{Präp} = 4 \end{cases}$$

$OZ_{Präp}$ = Ordnungszahl eines Präparates mit der die Präparate entsprechend TNC und Matchlevel geordnet werden können

$$OZ_{Präp} := \frac{TNC_{Präp}}{ZF_{Präp}}$$

$SL_{Single}$ = Shortlist der zu berücksichtigenden Präparate für Einzeltransplantate

$$SL_{Single} := \left\{ p \in \text{Pr}\ddot{a}p \left| \frac{OZ_{p}}{KG_{Pat}} \geq \frac{1}{kg} \wedge ML_{Präp} \geq 4 \right. \right\}$$

und die Standardordnungen der Präparate in der Shortlist nach Ordnung 1 oder Ordnung 2 erfolgt, wobei

$$\text{Ordnung 1 (SL)} := \begin{cases} p1 \in SL, p2 \in SL \left| \begin{array}{l} entweder \; ML_{p1} > ML_{p2} \\ oder \; ML_{p1} = ML_{p2} \wedge OZ_{p1} > OZ_{p2} \\ oder \; ML_{p1} = ML_{p2} \wedge OZ_{p1} = OZ_{p2} \wedge CD34_{p1} \geq CD34_{p2} \end{array} \right. \end{cases}$$

$$\text{Ordnung 2 (SL)} := \begin{cases} p1 \in SL, p2 \in SL \left| \begin{array}{l} entweder \; OZ_{p1} > OZ_{p2} \\ oder \; OZ_{p1} = OZ_{p2} \wedge ML_{p1} > ML_{p2} \\ oder \; OZ_{p1} = OZ_{p2} \wedge ML_{p1} = ML_{p2} \wedge CD34_{p1}^{+} \geq CD34_{p2}^{+} \end{array} \right. \end{cases}$$

wobei

Präp = Nabelschnurblutpräparat

Pat = Patient

$HLA_{Pat}$ = HLA Werte des Empfängers

$HLA_{Präp}$ = HLA Werte eines Präparates

$TNC_{Präp}$ = Anzahl der kernbehafteten Zellen eines Präparates

$KG_{Pat}$ = Körpergewicht des Patienten in kg

$CD34+_{Präp}$ = Anzahl der $CD34^+$ Zellen eines Präparates Parameter der Erfahrungs- und/oder Aufragedaten sind und

$ML_{Präp}$ = Matchlevel entsprechend der HLA Übereinstimmung zwischen Präparat und Patient

ist.

2. Verfahren nach Anspruch 1,
   **dadurch gekennzeichnet, dass** die potentiellen Nabelschnurblut-Präparate nach einem HLA-Match, einem Patientengewicht, einer Anzahl der kernhaltigen Zellen (TNC) und einer Anzahl der hämatopoetischen Zellen (CD34+) geordnet und ausgewählt werden, wobei zusätzlich vor und nach einer Transplantation und/oder einer Therapie von biologischen Zellen und/oder Geweben Metabolite der Nabelschnurblut-Präparate, des Empfängers und/oder des Spenders bestimmt werden und Genom-, Proteom-, Transkriptom- und/oder Epigenomanalysen durchgeführt werden.

3. Verfahren nach Anspruch 1 oder 2,
   **dadurch gekennzeichnet, dass**
   die Erfahrungsdaten der Nabelschnurblut-Präparate folgende Parameter umfassen:

   - Name und Kennung der lagernden Nabelschnurblut-Bank (NSB-Bank),
   - Status der lagernden NSB-Bank hinsichtlich internationaler Zertifizierungen, bevorzugt Fact,
   - Abwicklungszuverlässigkeit der NSB-Bank nach Klassifizierung,
   - Ansprechpartner an der betreffenden Bank mit Kontaktdaten,
   - Identifikationsnummer des Präparates,
   - Anamnese der Mutter, des Kindes und der Familie gemäß Anamnesebogen der Geburtsklinik,
   - Ethnie der Mutter, des Vaters und/oder des Kindes,
   - Geschlecht des Kindes,
   - Einlagerungszeitpunkt des Präparates,
   - Informationen zur Aufarbeitung des Präparates,
   - Blutgruppe des Präparates,
   - HLA-Typus des Präparates,
   - Zellzahl (TNC) des Präparates,
   - Zellzahl (CD34+) des Präparates,
   - Virusstatus des Präparates,
   - allele Ausprägungen des Präparates und/oder
   - Molekulare Diagnosen und Analysen des Genoms, Transkriptoms, Proteoms, Epigenoms und/oder Metaboloms

   wobei die Erfahrungsdaten auf einem Speichermedium und/oder Verarbeitungseinheit gespeichert werden.

4. Verfahren nach Anspruch 1 oder 2,
   **dadurch gekennzeichnet, dass**
   Metabolite aus dem Gewebe, den Schleimhäuten, der Lunge, der Leber, dem Blut, dem Darm, dem Kot, der Niere und/oder dem Urin bestimmt werden.

5. Verfahren nach mindestens einem der vorhergehenden Ansprüche,
   **dadurch gekennzeichnet, dass**
   die Recherche eine Ermittlung der zum Empfänger kompatiblen Nabelschnurblut-Präparate entsprechend folgender Ordnungs- und/oder Ausschlusskriterien umfasst:

   - Name und Kennung der Klinik bzw. des Transplantcenters,
   - Name des Koordinators und des behandelnden Arztes mit Kontaktdaten,
   - Status der Klinik hinsichtlich internationaler Zertifizierungen (z.B. Fact),
   - Durchschnittliche Anzahl an NSB-Transplantationen an der anfragenden Klinik in den letzten drei Jahren,

- Name des Patienten, Versicherungsnummer und weitere Abrechnungsdaten,
- Anamnese des Patienten,
- Indikation und Therapievorschlag des behandelnden Arztes,
- Dringlichkeit nach definierter Klassifizierung,
- HLA-Typus des Patienten,
- Blutgruppe des Patienten,
- Gewicht des Patienten,
- Ethnie des Patienten,
- Geschlecht des Patienten,
- Alter des Patienten,
- Bekannte allele Ausprägungen des Patienten und/oder Daten einer DNA-Typisierung und/oder
- Erst- oder Wiederholungsbehandlung,

wobei die Ordnungs- und/oder Ausschlusskriterien auf einem Speichermedium und/oder Verarbeitungseinheit gespeichert werden.

6. Verfahren nach mindestens einem der vorhergehenden Ansprüche,
**dadurch gekennzeichnet, dass**
folgende Ordnungskriterien und/oder Ausschlusskriterien herangezogen werden:

- Präparate mit einer CD34+ Zellzahl höher 10% der TNC Zellzahl,
- Ausschluss von Präparaten, bei denen im CA (Colony Assay) weniger als 75% der CD34+ Zellen überlebten bzw. aktiviert wurden,
- Blutgruppenidentität,
- Ethnische Identität,
- Geschlecht,
- Alter des Präparates,
- Akkreditierungsstandard und/oder
- Ranking der NSB-Bank.

7. Verfahren nach mindestens einem der vorhergehenden Ansprüche,
**dadurch gekennzeichnet, dass**
es zur Vermittlung von Doppel- oder Mehrfachtransplantationen (Multicord) eingesetzt wird.

8. Verfahren nach mindestens dem vorhergehenden Anspruch,
**dadurch gekennzeichnet, dass**
die Auswahl der Multicord-Präparate nach folgenden Ordnungskriterien erfolgt:

$ML_{P1P2}$ = Verträglichkeit von 2 Präparaten untereinander:

$$ML_{P1P2} := \begin{cases} 6: HLA_{\text{Präp1}} \text{ und } HLA_{\text{Präp2}} \text{ matchen in } 6 \text{ von } 6 \text{ Werten und Blutgruppenverträglichkeit} \\ 5: HLA_{\text{Präp1}} \text{ und } HLA_{\text{Präp2}} \text{ matchen in } 5 \text{ von } 6 \text{ Werten und Blutgruppenverträglichkeit} \\ 4: HLA_{\text{Präp1}} \text{ und } HLA_{\text{Präp2}} \text{ matchen in } 4 \text{ von } 6 \text{ Werten und Blutgruppenverträglichkeit} \\ \text{Präparat wird nicht berücksichtigt : sonst} \end{cases}$$

$GL_{Multi}$ = Grundliste zur Ermittlung der Auswahlliste für Multipräparate

$$GL_{Multi} := \left\{ p \in \text{Präp} \left| \frac{OZ_p}{KG_{Pat}} < \frac{1}{kg} \wedge ML_{\text{Präp}} \geq 4 \right. \right\}$$

SL$_{Multi}$ = Shortlist der zu berücksichtigenden Präparate für Multitransplantate

$$SL_{Multi} := \left\{ p1 \in GL_{Multi}, p2 \in GL_{Multi} \middle| ML_{p1p2} \geq 4 \wedge \frac{OZ_{p1} + OZ_{p2}}{KG_{Pat}} \geq 1 \right\}.$$

9. Verfahren nach einem oder mehreren der vorhergehenden Ansprüche,
   **dadurch gekennzeichnet, dass**
   für die Darstellung der über die Suchkriterien erlangten Ergebnisse eine Matrix verwendet und die Ergebnisse visuell darstellt.

10. Verfahren nach einem oder mehreren der vorhergehenden Ansprüche,
    **dadurch gekennzeichnet, dass**
    für die Beurteilung des Status der Ordnungskriterienermittlung mittels Statistiken über die zu erwartenden Kosten und die zu benötigende Zeit verwendet wird.

**Claims**

1. Method for the selection of at least one umbilical cord blood preparation that is suitable for the allogeneic transplant,
   **characterised in that**
   the method comprises the following steps:

   - Entry of experience data of umbilical cord blood preparations into a computer and storage on a storage medium,
   - Input of inquiry data of a potential recipient of the umbilical cord preparation of and storage of this on a storage medium,
   - Presetting of search criteria, in particular storage of the search criteria on a storage medium and/or a processing unit,
   - Search whereby the search comprises a comparison of the experience data with the inquiry data and whereby a collection centre, a storage site, a clinic, transplant centre and/or a research institution communicate with one another via cable and/or wireless connections on one or several processing units, in particular computer, medical systems, storage devices and/or special processors and via a network consisting of the several processing units through which data is exchanged, are connected with one another and
   - automatic evaluation of the search result whereby

   a ranking (SL$_{Single}$) of the identified preparations for the selection of at least one umbilical cord blood preparation that is suitable for the allogeneic transplant is produced based on the following settings:

$$ML_{Prep} = \begin{cases} 6 : HLA_{Prep} \text{ and } HLA_{Pat} \text{ match in } 6 \text{ out of } 6 \text{ values and blood group compatibility} \\ 5 : HLA_{Prep} \text{ and } HLA_{Pat} \text{ match in } 5 \text{ out of } 6 \text{ values and blood group compatibility} \\ 4 : HLA_{Prep} \text{ and } HLA_{Pat} \text{ match in } 4 \text{ out of } 6 \text{ values and blood group compatibility} \\ \text{Pr } eparation \text{ not included : other} \end{cases}$$

CF$_{Prep}$ = the cell factor defines the required cell count per kg of the patients weight in the case of a corresponding match level

$$\text{CF}_{\text{Prep}} := \begin{cases} 3 \times 10^7 : ML_{\text{Prep}} = 6 \\ 4 \times 10^7 : ML_{\text{Prep}} = 5 \\ 5 \times 10^7 : ML_{\text{Prep}} = 4 \end{cases}$$

$CN_{\text{Prep}}$ = Classification number of a preparation with which the preparations can be arranged in accordance with the TNC and match level

$$\text{CN}_{\text{Prep}} := \frac{TNC_{\text{Prep}}}{CF_{\text{Prep}}}$$

$SL_{\text{Single}}$ = Shortlist of the preparations to be considered for single transplants

$$\text{SL}_{\text{Single}} := \left\{ p \in \text{Prep} \middle| \frac{CN_p}{BW_{Pat}} \geq \frac{1}{kg} \wedge ML_{\text{Prep}} \geq 4 \right\}$$

and the standard classifications of the preparations in the shortlist according to classification 1 or classification 2 whereby

$$\text{Classification 1 (SL)} := \begin{cases} p1 \in SL, p2 \in SL \middle| \begin{array}{l} either\ ML_{p1} > ML_{p2} \\ or\ ML_{p1} = ML_{p2} \wedge CN_{p1} > CN_{p2} \\ or\ ML_{p1} = ML_{p2} \wedge CN_{p1} = CN_{p2} \wedge CD34_{p1} \geq CD34_{p2} \end{array} \end{cases}$$

$$\text{Classification 2 (SL)} := \begin{cases} p1 \in SL, p2 \in SL \middle| \begin{array}{l} either\ CN_{p1} > CN_{p2} \\ or\ CN_{p1} = CN_{p2} \wedge ML_{p1} > ML_{p2} \\ or\ CN_{p1} = CN_{p2} \wedge ML_{p1} = ML_{p2} \wedge CD34^+_{p1} \geq CD34^+_{p2} \end{array} \end{cases}$$

whereby

Prep = Umbilical cord blood preparation
Pat = Patient
$HLA_{Pat}$ = HLA values of the recipient
$HLA_{Prep}$ = HLA values of a preparation
$TNC_{Prep}$ = Number of nucleated cells of a preparation
$BW_{Pat}$ = Bodyweight of the patient in kg
$CD34+_{Prep}$ = number of $CD34^+$ cells of a preparation are the parameters of the experience and/or inquiry data, and
$ML_{Prep}$ = Match level according to the HLA match between the preparation and patient

2. Method according to claim 1,
   **characterised in that**

the potential umbilical cord blood preparations are arranged and selected according to an HLA match, patient weight, a number of nucleated cells (TNC) and a number of hematopoietic cells (CD34+), whereby metabolites of the umbilical cord blood preparations, of the recipient and/or the donor are determined before and after a transplant and/or a treatment of biological cells and/or tissues and gene, proteome, transcriptome and/or epigenome analyses are performed.

3. Method according to claim 1 or 2,
**characterised in that**
the experience data of the umbilical cord blood preparations comprises the following parameters:

- Name and identifier of the umbilical cord blood bank (UCB Bank) storing them,
- Status of the UCB bank storing them in terms of international certifications, preferably Fact,
- Reliability of processing of the UCB bank in accordance with classification,
- Contact of the bank concerned with contact details,
- Identification number of the preparation.
- Case history of the mother, the child and the family and the family history form of the maternity hospital,
- Ethnicity of the mother, the father and/or the child,
- Sex of the child,
- Storage time of the preparation,
- Information on the processing of the preparation,
- Blood type of the preparation,
- HLA type of the preparation,
- Number of cells (TNC) of the preparation,
- Number of cells (CD34+) of the preparation,
- Virus status of the preparation,
- allelic forms of the preparation and/or
- Molecular diagnoses and analyses of the genomes, transcriptome, proteome, epigenome and/or metabolom

whereby the experience data on is stored on a storage medium and/or processing unit.

4. Method according to claim 1 or 2,
**characterised in that**
metabolites from tissue, mucous membranes, lung, liver, blood, the colon, excrement, kidneys and/or urine are determined.

5. Method according to at least one of the preceding claims,
**characterised in that**
the search comprises a determination of the classification of the umbilical cord blood preparations that are compatible for the recipient in accordance with the following criteria of classification and/or exclusion criteria:

- the name and identifier of the clinic or the Transplant Center,
- Name of the coordinator and the physician with contact information,
- Status of the clinic with regard to international certifications (e.g. Fact),
- Average number of cord blood transplants at the requesting hospital in the last three years,
- Name of the patient, insurance number and other billing information,
- Case history of the patient,
- Indication and suggested treatment of the physician administering treatment,
- Urgency based on a defined classification,
- HLA type of the patient,
- Blood type of the patient,
- Weight of the patient,
- Ethnicity of the patient,
- Sex of the patient,
- Age of the patient,
- Known allelic characteristics of the patient and/or data of a DNA typing and/or
- Initial or repeat treatment,

whereby the classification and/or exclusion criteria are stored on a storage medium and/or processing unit.

**6.** Method according to at least one of the preceding claims,
**characterised in that**
the following criteria of classification and/or exclusion criteria are used:

- Preparations with CD34+ cell count that is higher than 10% of the TNC cell count,
- Exclusion of preparations in which less than 75% of the CD34+ cells survived or were activated in the CA (Colony Assay),
- Blood group identity,
- Ethnic identity,
- Gender,
- Age of the preparation,
- Accreditation standard and/or
- Ranking of UCB bank.

**7.** Method according to at least one of the preceding claims,
**characterised in that**
it is used to impart double or multiple transplants (multicord).

**8.** Method according to at least the preceding claim,
**characterised in that**
the multicord preparations are selected according to the following classification criteria:

$ML_{P1P2}$= tolerability of 2 preparations with one another:

$$ML_{P1P2} = \begin{cases} 6: HLA_{Prep1} \text{ and } HLA_{Prep2} \text{ match in } 6 \text{ out of } 6 \text{ values and blood group compatibility} \\ 5: HLA_{Prep1} \text{ and } HLA_{Prep2} \text{ match in } 5 \text{ out of } 6 \text{ values and blood group compatibility} \\ 4: HLA_{Prep1} \text{ and } HLA_{Prep2} \text{ match in } 4 \text{ out of } 6 \text{ values and blood group compatibility} \\ Pr \text{eparation not included : other} \end{cases}$$

$BL_{Multi}$ = basic list for determining the selection list for multipreparations

$$BL_{Multi} := \left\{ p \in Pr\,ep \left| \frac{CN_p}{BM_{Pat}} < \frac{1}{kg} \wedge ML_{Prep} \geq 4 \right. \right\}$$

$SL_{Multi}$ = Shortlist of the preparations to be considered for multiple transplants

$$SL_{Multi} := \left\{ p1 \in BL_{Multi}, p2 \in BL_{Multi} \left| ML_{p1p2} \geq 4 \wedge \frac{CN_{p1} + CN_{p2}}{BW_{Pat}} \geq 1 \right. \right\}.$$

**9.** Method according to one or several of the preceding claims,
**characterised in that**
a matrix is used for the display of the results achieved via the search criteria and the results are displayed visually.

**10.** Method according to one or several of the preceding claims,
**characterised in that**

for the evaluation of the status of the determination of the classification criteria by means of statistics concerning the costs to be expected and the time required is used.

**Revendications**

1. Procédé de sélection d'au moins une préparation de sang ombilical adaptée à la transplantation allogène, **caractérisé en que**
le procédé comprend les opérations suivantes :

   - saisie de données empiriques de préparations de sang ombilical dans un ordinateur et sauvegarde sur un support d'enregistrement,
   - saisie de données d'interrogation d'un récepteur potentiel de la préparation de sang ombilical et leur sauvegarde sur un support d'enregistrement,
   - paramétrage préalable de critères de recherche, en particulier de la sauvegarde des critères de recherche sur un support d'enregistrement et/ou une unité de traitement,
   - recherche, la recherche comprenant une comparaison des données empiriques avec les données d'interrogation et un centre de prélèvement, une unité d'entreposage, une clinique, un centre de transplantation et/ou un institut de recherche communiquant entre eux via des connexions câblées et/ou sans câble sur une ou plusieurs unités de traitement, en particulier un ordinateur, des systèmes médicaux, des dispositifs d'enregistrement et/ou des processeurs spéciaux et étant reliés entre eux via un réseau composé de plusieurs unités de traitement par lesquelles des données sont échangées
   - évaluation automatique des résultats de recherche,

   une liste de classement ($SL_{Single}$) des préparations identifiées étant établie pour la sélection d'au moins une préparation de sang ombilical adaptée à la transplantation allogène sur la base de l'ordre suivant :

$$ML_{Präp} = \begin{cases} 6 : HLA_{Pr\ddot{a}p} \; et \; HLA_{Pat} \; sont \; compatibles \; dans \; 6 \; valeurs \; sur \; 6 \; et \; compatibilité \; de \; groupe \; sanguin \\ 5 : HLA_{Pr\ddot{a}p} \; et \; HLA_{Pat} \; sont \; compatibles \; dans \; 5 \; valeurs \; sur \; 6 \; et \; compatibilité \; de \; groupe \; sanguin \\ 4 : HLA_{Pr\ddot{a}p} \; etd \; HLA_{Pat} \; sont \; compatibles \; dans \; 4 \; valeurs \; sur \; 6 \; et \; compatibilité \; de \; groupe \; sanguin \\ Pr \, \acute{e}paration \; non \; prise \; en \; compte : \sin on \end{cases}$$

$ZF_{Präp}$ = Le facteur cellulaire définit le nombre de cellules nécessaires par kg du poids du patient pour le nouveau correspondant de match

$$ZF_{Präp} := \begin{cases} 3 \times 10^7 : ML_{Pr\ddot{a}p} = 6 \\ 4 \times 10^7 : ML_{Pr\ddot{a}p} = 5 \\ 5 \times 10^7 : ML_{Pr\ddot{a}p} = 4 \end{cases}$$

$OZ_{Präp}$ = nombre d'ordre d'une préparation avec lequel les préparations peuvent être classées conformément au TNC et au niveau de compatibilité

$$OZ_{Präp} := \frac{TNC_{Pr\ddot{a}p}}{ZF_{Pr\ddot{a}p}}$$

SL$_{Single}$ = La courte liste des implantations individuelles pour les préparations à prendre en compte

$$SL_{Single} := \left\{ p \in Pr\,\ddot{a}p \left| \frac{OZ_p}{KG_{Pat}} \geq \frac{1}{kg} \wedge ML_{Pr\ddot{a}p} \geq 4 \right. \right\}$$

et les nombres standard des préparations dans la courte liste s'effectue selon l'ordre 1 ou 2,

$$Ordre\ 1\ (SL) := \left\{ p1 \in SL, p2 \in SL \left| \begin{array}{l} soit\ ML_{p1} > ML_{p2} \\ ou\ ML_{p1} = ML_{p2} \wedge OZ_{p1} > OZ_{p2} \\ ou\ ML_{p1} = ML_{p2} \wedge OZ_{p1} = OZ_{p2} \wedge CD34_{p1} \geq CD34_{p2} \end{array} \right. \right.$$

$$Ordre\ 2\ (SL) := \left\{ p1 \in SL, p2 \in SL \left| \begin{array}{l} soit\ OZ_{p1} > OZ_{p2} \\ ou\ OZ_{p1} = OZ_{p2} \wedge ML_{p1} > ML_{p2} \\ ou\ OZ_{p1} = OZ_{p2} \wedge ML_{p1} = ML_{p2} \wedge CD34^+_{p1} \geq CD34^+_{p2} \end{array} \right. \right.$$

Präp = Préparation de sang ombilical
Pat = Patient
HLA$_{Pat}$= Valeur HLA du récepteur
HLA$_{Präp}$ = Valeur HLA d'une préparation
TNC$_{Präp}$ = Nombre de cellules nuclées d'une préparation
KG$_{Pat}$= Poids du corps du patient en kg
CD34+$_{Präp}$= Nombre des CD34$^+$ cellules d'une préparation étant les paramètres des données empiriques et/ou d'interrogation et
ML$_{Präp}$= Niveau de compatibilité conformément à la concordance HLA entre la préparation et le patient

2. Procédé selon la revendication 1
**caractérisé en ce que** les préparations de sang ombilical potentielles sont classées et sélectionnées selon un match HLA, un poids du patient, un nombre de cellules nuclées (TNC) et une nombre de cellules hématopoïétiques (CD34+), de plus avant et après une transplantation et/ou une thérapie de cellules biologiques et/ou de tissus, des métabolites des préparation de sang ombilical, du récepteur et/ou du donneur étant déterminées et des analyses de génomes, de protéomes, de transcriptomes et/ou d'épigénomes étant effectuées.

3. Procédé selon la revendication 1 ou 2
**caractérisé en que**
les données empiriques des préparations de sang ombilical contiennent les paramètres suivants :

   - nom et identification de la banque des préparations de sang ombilical (banque PSO),
   - statut de la banque PSO à entreposer concernant les certifications internationales, de préférence Fact,
   - fiabilité de la procédure de la banque PSO selon classification,
   - interlocuteur de la banque concernée avec données de contact,
   - numéro d'identification de la préparation,
   - anamnèse de la mère, de l'enfant et de la famille conformément au questionnaire d'anamnèse de la maternité,
   - ethnie de la mère, du père et/ou de l'enfant,
   - sexe de l'enfant,
   - date du stockage de la préparation,
   - information sur la réalisation de la préparation,
   - groupe sanguin de la préparation,
   - type HLA de la préparation,
   - nombre de cellules (TNC) de la préparation,
   - nombre de cellules (CD34+) de la préparation,

- statut de virus de la préparation,
- caractéristiques allèles de la préparation et/ou
- diagnostics moléculaires et analyses du génome, du transcriptome, du protéomes, de l'épigénome et/ou du métabolome

les données empiriques étant stockées sur un support de données et/ou une unité de traitement.

4. Procédé selon la revendication 1 ou 2
   **caractérisé en que**
   des métabolites sont déterminées à partir du tissu, des muqueuses, du poumon, du foie, de l'intestin, des excréments, du rein et/ou de l'urine.

5. Procédé selon au moins l'une des revendications précédentes
   **caractérisé en que**
   la recherche comprend la détermination des préparations de sang ombilical compatibles au récepteur conformément aux critères d'ordre et/ou d'exclusion suivants :

   - nom et identification de la clinique ou du centre de transplantation,
   - nom du coordinateur et du médecin traitant avec données de contact,
   - statut de la clinique concernant les certifications internationales, (p.ex. Fact),
   - nombre moyen de transplantations PSO dans la clinique en question ces trois dernières années,
   - nom du patient, numéro d'assurance et autres données de décompte,
   - anamnèse du patient,
   - indication et proposition de thérapie du médecin traitant,
   - urgence selon la classification définie,
   - type HLA du patient,
   - groupe sanguin du patient,
   - poids du patient,
   - ethnie du patient,,
   - sexe du patient,
   - âge du patient,
   - caractéristiques allèles connues du patient et/ou données d'une typage ADN et/ou
   - premier traitement ou traitement répété,

   les critères d'ordre et/ou d'exclusion étant stockés sur un support de données et/ou une unité de traitement.

6. Procédé selon au moins l'une des revendications précédentes
   **caractérisé en que**
   l'on a recours aux critères d'ordre et/ou aux critères d'exclusion suivants :

   - préparations avec un nombre de cellules supérieur à 10% et au nombre de cellules TNC,
   - exclusion de préparations où dans le CA (Colony Assay) moins de 75% des cellules CD34+ survivent ou ont été activées,
   - identité du groupe sanguin,
   - identité ethnique,
   - sexe,
   - âge de la préparation,
   - standard d'accréditation et/ou und/oder
   - classement de la banque PSO.

7. Procédé selon au moins l'une des revendications précédentes
   **caractérisé en que**
   il est utilisé pour déterminer les transplantations doubles ou multiples (multicord).

8. Procédé selon au moins l'une des revendications précédentes
   **caractérisé en que**
   la sélection des préparations multicord s'effectue selon les critères d'ordre suivants :

$ML_{P1P2}$= compatibilité de 2 préparations entre elles:

$$ML_{P1P2} := \begin{cases} 6 : HLA_{Pr\ddot{a}p} \text{ } et \text{ } HLA_{Pat} \text{ } sont \text{ } compatibles \text{ } dans \text{ } 6 \text{ } valeurs \text{ } sur \text{ } 6 \text{ } et \text{ } compatibilité \text{ } de \text{ } groupe \text{ } sanguin \\ 5 : HLA_{Pr\ddot{a}p} \text{ } et \text{ } HLA_{Pat} \text{ } sont \text{ } compatibles \text{ } dans \text{ } 5 \text{ } valeurs \text{ } sur \text{ } 6 \text{ } et \text{ } compatibilité \text{ } de \text{ } groupe \text{ } sanguin \\ 4 : HLA_{Pr\ddot{a}p} \text{ } etd \text{ } HLA_{Pat} \text{ } sont \text{ } compatibles \text{ } dans \text{ } 4 \text{ } valeurs \text{ } sur \text{ } 6 \text{ } et \text{ } compatibilité \text{ } de \text{ } groupe \text{ } sanguin \\ Pr\text{ }éparation \text{ } non \text{ } prise \text{ } en \text{ } compte : \sin on \end{cases}$$

$GL_{Multi}$ = liste de base pour déterminer la liste de sélection des multipréparations

$$GL_{Multi} := \left\{ p \in \Pr \check{S}p \left| \frac{OZ_p}{KG_{Pat}} < \frac{1}{kg} \wedge ML_{\Pr \check{S}p} \geq 4 \right. \right\}$$

$SL_{Multi}$ = courte liste des préparations à prendre pour les multigreffes

$$SL_{Multi} := \left\{ p1 \in GL_{Multi}, p2 \in GL_{Multi} \left| ML_{p1p2} \geq 4 \wedge \frac{OZ_{p1} + OZ_{p2}}{KG_{Pat}} \geq 1 \right. \right\}.$$

9. Procédé selon l'une ou plusieurs revendications précédentes,
   **caractérisée en que**
   pour la représentation des résultats obtenus par les critères de recherche, on utilise une matrice et on représente les résultats visuellement.

10. Procédé selon l'une ou plusieurs revendications précédentes
    **caractérisé en que**
    on utilise pour l'évaluation du statut de la détermination de critères d'ordre via des statistiques sur les coûts à attendre et le temps nécessaire.

Fig. 1:

Fig. 2:

**Datenaufbereitung**

NSB Präparate Upload: NSB Daten (Unit Reports) werden in NSB Bank erfasst und in das System eingespielt

Erweiterung der NSB Präparat Verträglichkeitsmatrix: Daten aller neuen NSB Präparate werden mit den im System vorhandenen NSB Präparate auf Kompatibilität geprüft und Verträglichkeitsmatrix wird erweitert

**Voreinstellung der Suchkriterien**

Festlegung der Standard-Suchprofile für Kliniken und Ärzte

Individualisierung der Ordnungs und Ausschlusskriterien nach Koordinatorpräferenzen

**Transplantat Suche**

Neuer Patient wird erfasst, zusätzlice NSB Präparate können eingespielt, Suchparameter können fallspezifisch angepasst werden

Basic Serach: Ermittlung aller zum Patienten kompatiblen Präparate entsprechend der voreingestellten Ordungs- und Auschlusskriterien (Long List)

**Advanced Search**

Ermittlung der möglichen Singletransplantate (Shortlist SingleCord)

Ermittlung der möglichen Multitransplantate (Shortlist MultiCord)

Berechung des Budgets und der benötigten Zeit sowie der Ergebnisqualität pro Transplant aufgrund der noch durchzuführenden Tests etc.

**Lösungsgenerierung**

Absolute Reihung aller Shortlistpräparate nach vorgegebenen Präferenzen

Betätigung / Korrektur des Lösungsvorschlages durch den Arzt

Erzeugung der Patientenakte: Laufzettel, Unit Report, Entscheidungsvorlage, Patientendokumentation

Optional: Comparison View für selektierte Präparate und manuelle Auswahl

**Bestellabwicklung & Nachverfolung**

Bestellung des NSB Präparates

Reservierung von Präparaten und Festlegung von Backup-Präparaten

Bestellauslösung mit Versand und Fakturierung

Eingangskontrolle der NSB-Präparate

Nachverfolgung des Patienten (Follow-Up)

Abwicklung von Verifikationsaufträgen (DNA Proben, High Resolution Typisierung, ...)

Fig. 3:

# Fig. 4:

## Diagnose, Nachfolgeuntersuchung und editierte Entwicklungsdaten

┈┈┈▶ = Datentransfer,  ──────▶ = Gewebe, Blut, DNA Probentransfer

CBU: "Cord Blood Unit" (Englisch),
Nabelschnurbluteinheit (Deutsch);
CBB: "Cord Blood Bank" (Englisch),
Nabelschnurblutbank (Deutsch)

EP 2 238 553 B1

**EP 2 238 553 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20020132343 A1
- US 20020168639 A1
- GB 2407193 A
- US 20040121369 A1

- WO 02077640 A2
- US 20080014174 A1
- DE 60030978 T2
- WO 2005097190 A